(19) Europäisches Patentamt
European Patent Office
Office européen des brevets

(11) EP 2 586 772 A1

(12) **EUROPEAN PATENT APPLICATION**
published in accordance with Art. 153(4) EPC

(43) Date of publication:
**01.05.2013 Bulletin 2013/18**

(21) Application number: **11798156.3**

(22) Date of filing: **22.06.2011**

(51) Int Cl.:
***C07D 217/18*** (2006.01) ***A61K 31/472*** (2006.01)
***A61P 11/02*** (2006.01) ***A61P 11/06*** (2006.01)
***A61P 17/04*** (2006.01) ***A61P 37/08*** (2006.01)
***A61P 43/00*** (2006.01) ***C07D 217/20*** (2006.01)

(86) International application number:
**PCT/JP2011/064208**

(87) International publication number:
**WO 2011/162274 (29.12.2011 Gazette 2011/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **23.06.2010 JP 2010142187**

(71) Applicant: **Taisho Pharmaceutical Co., Ltd.**
**Tokyo 170-8633 (JP)**

(72) Inventors:
- **TAKAYAMA Tetsuo**
  **Tokyo 170-8633 (JP)**
- **KAWAMURA Madoka**
  **Tokyo 170-8633 (JP)**
- **WAKASUGI Daisuke**
  **Tokyo 170-8633 (JP)**
- **NISHIKAWA Rie**
  **Tokyo 170-8633 (JP)**
- **SEKIGUCHI Yoshinori**
  **Tokyo 170-8633 (JP)**

(74) Representative: **HOFFMANN EITLE**
**Patent- und Rechtsanwälte**
**Arabellastrasse 4**
**81925 München (DE)**

(54) **ISOQUINOLINE DERIVATIVE**

(57) A compound represented by formula (I) or a pharmaceutically acceptable salt thereof has an effect of inhibiting CRTH2 and, therefore, is useful as a pharmaceutical.

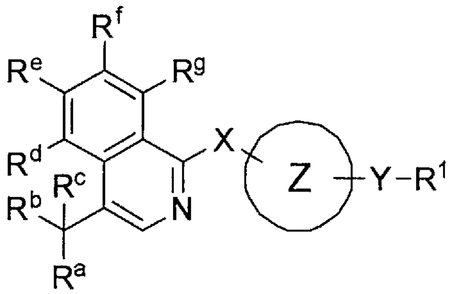

(I)

EP 2 586 772 A1

## Description

Technical Field

The present invention relates to a compound having an inhibitory effect on CRTH2 (Chemoattractant Receptor-homologous molecule expressed on Th2 cells), and pharmaceutical preparations containing the compound as an active ingredient.

Background Art

CRTH2 is a G-protein coupled 7th transmembrane domain molecule cloned by Nagata et al. in 1999 as a molecule expressed selectively on Th2 cells (see Non Patent Document 1).

It has been reported that the Th2 cell is one form of activated T cells and induces production of IgE from B cells via production of cytokines such as IL-4, IL-5, and IL-13 (see Non Patent Document 2). Furthermore, it has been reported that the cytokines induce the activation of eosinophil and basophil (see Non Patent Documents 3 and 4). From the above reports, it has been believed that the Th2 cells are strongly involved in the formation of pathologic conditions of allergic diseases such as asthma, allergic rhinitis, and atopic dermatitis directly or indirectly via other cells or factors (see Non Patent Document 5).

Because CRTH2 is cloned as a molecule expressed selectively on the Th2 cell as mentioned above, and also, it has relatively high homology to a chemokine receptor (see Non Patent Document 6), it has been assumed that CRTH2 is involved in immune responses or immune-related disorders. Thereafter, it has been revealed that CRTH2 is expressed in eosinophil and basophil in addition to the Th2 cell, and that the ligand is PGD2 and the action thereof induces a cell migration reaction and the like (see Non Patent Document 7). In particular, it has been suggested that CRTH2 is involved in allergic diseases.

In addition to such in vitro tests, in exacerbation of symptoms in an asthma model by a CRTH2-specific ligand and in a dermatitis model (see Non Patent Document 8), suppression of symptoms in dermatitis in a CRTH2 defective mouse (see Non Patent Document 9), increase in expression of CRTH2 in human patients with allergic rhinitis (Non Patent Document 10), and the like, the possibility that CRTH2 is involved in allergic diseases such as asthma, atopic dermatitis, and allergic rhinitis has been reported. From such information, the possibility of creation of therapeutic agents for the above-mentioned diseases, which have a mechanism of inhibiting CRTH2, has been suggested.

Conventionally, as CRTH2 inhibitors, indolyl acetic acid derivatives (see Patent Document 1), phenoxy acetic acid derivatives (see Patent Document 2), pyrimidinyl acetic acid derivative (see Patent Document 3) and the like have been reported. However, a compound having the structure of the present invention has not been disclosed. Furthermore, although a compound of which the structure is similar to that of the compound of the present invention has been reported, there is neither description nor suggestion that such compounds have a CRTH2 inhibitory effect (see Patent Document 4).

Prior Art Document

Patent Document

Patent Document 1: WO2005/019171
Patent Document 2: WO2005/115382
Patent Document 3: WO2004/096777
Patent Document 4: W02004/101528


Non Patent Document

Non Patent Document 1: Nagata. et al. J. Immunol. 162. 1278. 1999
Non Patent Document 2: Del Prete. et al. Allergy. 47. 450. 1992
Non Patent Document 3: Pope. et al. J. Allergy. Clin. Immunol. 108. 594. 2001
Non Patent Document 4: Min. et al. Curr. Opin. Hematol. 15. 59. 2008
Non Patent Document 5: Broide. et al. J. Allergy. Clin. Immunol. 108 (2 suppl.). S65. 2001
Non Patent Document 6: Abe. et al. Gene. 227. 71. 1999
Non Patent Document 7: Hirai. et al. J. Exp. Med. 193. 225. 2001

Non Patent Document 8: Shiraishi. et al. J. Pharmacol. Exp. Ther. 312. 954. 2005
Non Patent Document 9: Satoh. et al. J. Immunol. 177. 2621. 2006
Non Patent Document 10: Kano. et al. Am. J. Rhinol. 20. 342. 2006

Summary of Invention

Problem to be Solved by the Invention

An object of the present invention is to provide a compound having an inhibitory effect on CRTH2 and being useful as pharmaceutical preparations.

Means for Solving the Problem

The present inventors have keenly carried out investigations for achieving the above-mentioned objects, and resulted in finding that novel isoquinoline derivatives achieve the above-mentioned object and have arrived at the present invention.

That is to say, the present invention is

(1) a compound represented by formula (I):

[Ka 1]

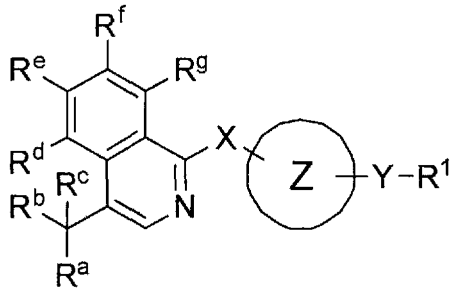

(I)

or a pharmaceutically acceptable salt thereof.

In the formula,

$R^1$ represents a $C_{1-6}$ alkyl group, a $C_{2-6}$ alkenyl group, a $C_{3-6}$ cycloalkyl group, a $C_{3-6}$ cycloalkenyl group, an adamantyl group, an indanyl group, a tetrahydronaphthyl group, a tetrahydroindolyl group, a tetrahydropyranyl group, a morpholinyl group, a phenyl group, a naphthyl group, or an aromatic heterocyclic group, wherein the phenyl group, the naphthyl group, and the aromatic heterocyclic group may be substituted with 1 to 5 substituent(s) selected from the group consisting of a $C_{1-6}$ alkyl group, a $C_{2-6}$ alkenyl group, a $C_{3-6}$ cycloalkyl group, a halogen atom, a $C_{1-6}$ alkoxy group, a hydroxy group, a $C_{1-6}$ alkylthio group, a $C_{1-6}$ haloalkyl group, a $C_{1-6}$ haloalkoxy group, a $C_{1-6}$ haloalkylthio group, a cyano group, a nitro group, a guanidino group, a $C_{1-6}$ alkylsulfonyl group, a carboxy group, a $C_{2-7}$ alkoxycarbonyl group, a $C_{2-7}$ alkanoyloxy group, a phenyl group, a benzoyl group, a phenoxy group, a pyrrolyl group, a thienyl group, an imidazolyl group, a thiadiazolyl group, a morpholino group, the formula: $-NR^5R^6$, the formula: $-SO_2NR^7R^8$, the formula: $-NR^9SO_2R^{10}$, the formula: $-CONR^{11}R^{12}$, and the formula: $-NR^{13}COR^{14}$, wherein $R^5$, $R^6$, $R^7$, $R^8$, $R^9$, $R^{10}$, $R^{11}$, $R^{12}$, $R^{13}$, and $R^{14}$ each independently represent a hydrogen atom or a $C_{1-6}$ alkyl group;

X represents an oxygen atom, a sulfur atom, the formula: $-CR^hR^i-$, the formula: -CO-, the formula: $-NR^2-$, or formula (II):

[Ka 2]

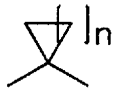

(II)

wherein $R^h$ and $R^i$ each independently represent a hydrogen atom, a $C_{1-6}$ alkyl group, a halogen atom or a $C_{1-6}$ haloalkyl group;
$R^2$ represents a hydrogen atom or a $C_{1-6}$ alkyl group;
n represents an integer of 1 to 4;
Y represents a single bond, the formula: $-NR^3CO-W-$, the formula: $-NR^3CO-W-O-$, the formula: $-NR^3CO_2-W-$, the formula: $-NR^3-W-$, the formula: $-NR^3SO_2-W-$, the formula: $-NR^3CONR^4-W-$, the formula: $-NR^3CO-W-NR^4SO_2-$, the formula: $-SO_2NR^3-W-$, the formula: $-CH_2-W-$, the formula: $-CONR^3-W-$, the formula: $-CONR^3-W-O-$, the formula: $-CH_2-O-W-$, the formula: $-CH_2NR^3-W-$, the formula: $-CONR^3-W-NR^4CO-$, the formula: -O-W-, or the formula: -O-W-O-, wherein $R^3$ and $R^4$ each independently represent a hydrogen atom or a $C_{1-6}$ alkyl group, W is a single bond, a $C_{1-6}$ alkylene group, a $C_{2-6}$ alkylene group including a carbon atom that is also a member of a $C_{3-6}$ cycloalkyl ring, a $C_{2-6}$ alkenylene group, or a $C_{3-6}$ cycloalkylene group (provided that, when Y is the formula: $-CONR^3-W-NR^4CO-$ or the formula: -O-W-O-, W is not a single bond);
Z represents a benzene ring, a pyrimidine ring, or a pyrazine ring;
said benzene ring, pyrimidine ring, and pyrazine ring may be substituted with 1 to 4 substituent(s) selected from the group consisting of a $C_{1-6}$ alkyl group, a $C_{2-6}$ alkenyl group, a $C_{3-6}$ cycloalkyl group, a halogen atom, a $C_{1-6}$ alkoxy group, a hydroxy group, a $C_{1-6}$ alkylthio group,
a $C_{1-6}$ haloalkyl group, a $C_{1-6}$ haloalkoxy group, a $C_{1-6}$haloalkylthio group, a cyano group, a nitro group, a $C_{1-6}$ alkylsulfonyl group, a carboxy group, the formula: $-NR^{22}R^{23}$ the formula: $-SO_2NR^{24}R^{25}$, the formula: $-NR^{26}SO_2R^{27}$, the formula: $-CONR^{28}R^{29}$, and the formula: $-NR^{30}COR^{31}$, wherein $R^{22}$, $R^{23}$, $R^{24}$, $R^{25}$, $R^{26}$, $R^{27}$, $R^{28}$, $R^{29}$, $R^{30}$, and $R^{31}$ each independently represent a hydrogen atom or a $C_{1-6}$ alkyl group (provided that, when Z is an unsubstituted benzene ring, unsubstituted pyrimidine ring, or unsubstituted pyrazine ring, X is the formula: $-CR^hR^i-$, or formula (II), wherein at least one of $R^h$ and $R^i$ represents a $C_{1-6}$ alkyl group, a halogen atom, or a $C_{1-6}$ haloalkyl group);
$R^a$ is a carboxy group, a carbamoyl group, a tetrazolyl group, or the formula: -CONHOH;
$R^b$ and $R^c$ each independently represent a hydrogen atom, a halogen atom, or a $C_{1-6}$ alkyl group; and
$R^d$, $R^e$, $R^f$ and $R^g$ each independently represent a hydrogen atom, a halogen atom, a $C_{1-6}$ alkyl group, or a $C_{1-6}$ alkoxy group (provided that the compound is not {1-[2-fluoro-5-(propan-2-yloxy)benzyl] -6,7-dimethoxyisoquinolin-4-yl}acetic acid, 2-[1-(2-fluoro-5-methoxybenzoyl)-6,7-dimethoxyisoquinolin-4-yl)acetamide, {1-[3-fluoro-5-(propan-2-yloxy)benzoyl]-6,7-dimethoxyisoquinolin-4-yl}acetic acid, 2-{1-[3-fluoro-5-(propan-2-yloxy)benzoyl]-6,7-dimethoxyisoquinolin-4-yl}propanoic acid, 2-{1-[2-fluoro-5-(propan-2-yloxy)benzoyl]-6,7-dimethoxyisoquinolin-4-yl}-4-methylpentanoic acid, 2-{1-[2-fluoro-5-(propan-2-yloxy)benzoyl] -6,7-dimethoxyisoquinolin-4-yl}propanoic acid, {1-[2-fluoro-5-(propan-2-yloxy) benzoyl] -6,7-dimethoxyisoquinolin-4-yl}acetic acid, or [6,7-dimethoxy-4-(1H-tetrazol-5-ylmethyl)isoquinolin-1-yl](2-fluoro-5-methoxyphenyl)methanone);
or a pharmaceutically acceptable salt thereof

(2) The compound or a pharmaceutically acceptable salt thereof as stated in (1), wherein $R^d$, $R^e$, $R^f$ and $R^g$ each independently represent a hydrogen atom, a halogen atom, a $C_{1-6}$ alkyl group, or a $C_{1-6}$ alkoxy group (except the compound or a pharmaceutically acceptable salt thereof in which both $R^d$ and $R^g$ are hydrogen atoms and both $R^e$ and $R^f$ are $C_{1-6}$ alkoxy groups).

(3) The compound or a pharmaceutically acceptable salt thereof according to (1) or (2), wherein X is the formula: $-CR^hR^i-$, wherein $R^h$ is a hydrogen atom, a $C_{1-6}$ alkyl group, or a halogen atom; and $R^i$ is a $C_{1-6}$ alkyl group, or a halogen atom.

(4) The compound or a pharmaceutically acceptable salt thereof according to any one of (1) to (3), wherein
Z is a benzene ring substituted with a $C_{1-6}$ alkyl group, a halogen atom, a $C_{1-6}$ alkoxy group, or a $C_{1-6}$ haloalkyl group.

(5) The compound or a pharmaceutically acceptable salt thereof according to any one of (1) to (4), wherein
$R^1$ is a phenyl group, which may be substituted with a halogen atom;
Y is the formula: $-CONR^3-W-$;
W is a $C_{1-6}$ alkylene group;
$R^a$ is a carboxy group;

$R^b$ and $R^c$ are each a hydrogen atom, and
$R^d$, $R^e$, $R^f$ and $R^g$ are each a hydrogen atom.

(6) A preventive or a remedy for asthma, atopic dermatitis and allergic rhinitis, comprising the compound or a pharmaceutically acceptable salt thereof according to any one of (1) to (5) as an active ingredient.

Advantageous Effects of the Invention

The compound of the present invention has an inhibitory effect on CRTH2.

Mode for Carrying Out the Invention

In the present invention, the $C_{1-6}$ alkyl group refers to a linear or branched alkyl group having 1 to 6 carbon atoms, and examples thereof include a methyl group, an ethyl group, a n-propyl group, an isopropyl group, a n-butyl group, an isobutyl group, a tert-butyl group, a sec-butyl group, a n-pentyl group, an isopentyl group, a neopentyl group, a tert-pentyl group, and a n-hexyl group.

The $C_{2-6}$ alkenyl group refers to a linear or branched alkenyl group having 2 to 6 carbon atoms, and examples thereof include a vinyl group, an allyl group, a 1-propenyl group, an isopropenyl group, a 1-butenyl group, a 2-butenyl group, a 3-butenyl group, a 1-pentenyl group, a 2-pentenyl group, a 1-hexenyl group, and a 1,3-butadienyl group.

The $C_{1-6}$ alkylene group refers to a linear or branched alkylene group having 1 to 6 carbon atoms, and examples thereof include a methylene group, an ethylene group, a trimethylene group, a tetramethylene group, a pentamethylene group, a hexamethylene group, an ethylidene group, a dimethyl methylene group, and a methyl ethylene group.

Examples of the $C_{2-6}$ alkylene group including a carbon atom that is also a member of a $C_{3-6}$ cycloalkyl ring include a 1,1-ethylene ethylene group, a 1,1-trimethylene ethylene group, a 1,1-tetramethylene ethylene group, a 1, 1-pentamethylene ethylene group, a 1,1-ethylene trimethylene group, and a 2,2-ethylene tirmethylene group. Each of the above-mentioned groups is shown below.

[Ka 3]

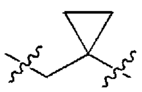 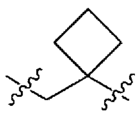 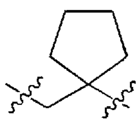 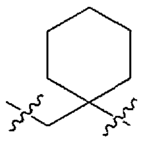 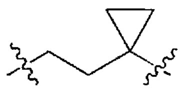 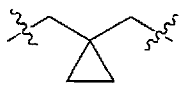

The $C_{2-6}$ alkenylene group refers to a linear or branched alkenylene group having 2 to 6 carbon atoms, and examples thereof include an ethenylene group, a propenylene group, and a methylethenylene group.

The $C_{3-6}$ cycloalkyl group refers to a cyclopropyl group, a cyclobutyl group, a cyclopentyl group, and a cyclohexyl group.

The $C_{3-6}$ cycloalkenyl group refers to a cycloalkenyl group having 3 to 6 carbon atoms, and examples thereof include a cyclobutenyl group, a cyclopentenyl group, a cyclohexenyl group, a cyclopentadienyl group, and a cyclohexadienyl group.

The $C_{3-6}$ cycloalkylene group refers to a cycloalkylene group having 3 to 6 carbon atoms, and examples thereof include a cyclopropanc-1,1-diyl group, a cyclobutane-1,1-diyl group, a cyclopentane-1,1-diyl group, a cyclohexane-1,1-diyl group, and a cyclohexane-1,4-diyl group.

The aromatic heterocyclic group refers to a monocyclic aromatic heterocyclic group or a condensed ring aromatic heterocyclic group including one or two heteroatom(s) selected from an oxygen atom, a nitrogen atom, and a sulfur atom in its ring, and examples thereof include a pyridyl group, a pyrimidyl group, a pyridazyl group, a pyrazinyl group, an oxazolyl group, a thiazolyl group, an isoxazolyl group, an isothiazolyl group, an indolyl group, a benzofuranyl group, a benzothienyl group, an imidazolyl group, a thienyl group, a furyl group, a pyrazolyl group, a pyrrolyl group, a quinoxalyl group, a quinolyl group, an isoquinolyl group, a quinazolyl group, a cinnolinyl group, a pyrrolopyridyl group, a naphthyridyl group, an imidazopyridyl group, an indazolyl group, a benzothiazolyl group, a benzoimidazolyl group, and a benzooxazolyl group.

The halogen atom refers to a fluorine atom, a chlorine atom, a bromine atom, and an iodine atom.

The $C_{1-6}$ alkoxy group refers to a linear or branched alkoxy group having I to 6 carbon atoms, and examples thereof include a methoxy group, an ethoxy group, a n-propoxy group, an isopropoxy group, a n-butoxy group, an isobutoxy group, a tert-butoxy group, a sec-butoxy group, a n-pentyloxy group, an isopentyloxy group, a neopentyloxy group, a tert-pentyloxy group, and a n-hexyloxy group.

The $C_{1-6}$ alkylthio group refers to a linear or branched alkylthio group having 1 to 6 carbon atoms, and examples thereof include a methylthio group, an ethylthio group, a n-propylthio group, an isopropylthio group, a n-butylthio group, an isobutylthio group, a tert-butylthio group, a sec-butylthio group, a n-pentylthio group, an isopentylthio group, a neopentylthio group, a tert-pentylthio group, and a n-hexylthio group.

The $C_{1-6}$ haloalkyl group refers to a linear or branched alkyl group having 1 to 6 carbon atoms, substituted with halogen atoms, in which the preferable number of halogen atoms is 3 to 5. Examples thereof include a trifluoromethyl group and a pentafluoroethyl group.

The $C_{1-6}$ haloalkylthio group refers to a linear or branched alkylthio group having 1 to 6 carbon atoms, substituted with halogen atoms, in which the preferable number of halogen atoms is 3 to 5. Examples thereof include a trifluoromethylthio group and a pentafluoroethylthio group.

The $C_{1-6}$ haloalkoxy group refers to a linear or branched alkoxy group having 1 to 6 carbon atoms, substituted with halogen atoms, in which the preferable number of halogen atoms is 3 to 5. Examples thereof include a trifluoromethoxy group and a pentafluoroethoxy group.

The $C_{1-6}$ alkylsulfonyl group refers to a linear or branched alkylsulfonyl group having 1 to 6 carbon atoms, and examples thereof include a methylsulfonyl group, an ethylsulfonyl group, a n-propylsulfonyl group, an isopropylsulfonyl group, a n-butylsulfonyl group, an isobutylsulfonyl group, a tert-butylsulfonyl group, a sec-butylsulfonyl group, a n-pentylsulfonyl group, an isopentylsulfonyl group, a neopentylsulfonyl group, a tert-pentylsulfonyl group, and a n-hexylsulfonyl group.

The $C_{2-7}$ alkoxycarbonyl group refers to a linear or branched alkoxycarbonyl group having 2 to 7 carbon atoms, and examples thereof include a methoxycarbonyl group, an ethoxycarbonyl group, a n-propoxycarbonyl group, an isopropoxycarbonyl group, a n-butoxycarbonyl group, an isobutoxycarbonyl group, a tert-butoxycarbonyl group, a sec-butoxycarbonyl group, a n-pentyloxycarbonyl group, an isopentyloxycarbonyl group, a neopentyloxycarbonyl group, a tert-pentyloxycarbonyl group, and a n-hexyloxycarbonyl group.

The $C_{2-7}$ alkanoyloxy group refers to a linear or branched alkanoyloxy group having 2 to 7 carbon atoms, and examples thereof include an acetoxy group, propanoyloxy group, a n-butanoyloxy group, and an isobutyroyloxy group.

The pharmaceutically-acceptable salt refers to a salt with an alkali metal, an alkali earth metal, ammonium, alkylammonium, or the like, or a salt with a mineral acid or an organic acid. Examples thereof include sodium salts, potassium salts, calcium salts, ammonium salts, aluminum salts, triethylammonium salts, acetates, propionates, butyrates, formates, trifluoroacetates, maleates, tartarates, citrates, stearates, succinates, ethylsuccinates, lactobionates, gluconates, glucoheptonates, benzoates, methanesulfonates, ethanesulfonates, 2-hydroxyethanesulfonates, benzenesulfonates, para-toluenesulfonates, laurylsulfates, malates, aspartates, glutamates, adipates, salts with cysteine, salts with N-acetylcysteines, hydrochlorides, hydrobromides, phosphates, sulfates, hydroiodides, nicotinates, oxalates, picrates, thiocyanates, undecanates, salts with acrylic acid polymers, and salts with carboxyvinyl polymers.

The compound of the present invention or a pharmaceutically acceptable salt thereof may be present as a solvate. Examples of the solvate may include hydrates of the compounds, and hydrates of the pharmaceutically acceptable salts of the compounds. They are all encompassed in the present invention.

When the compounds of the present invention are used as pharmaceutical preparations, the compounds of the present invention may be formulated, by the addition of commonly used excipients, extenders, pH adjusting agents, solubilizers, and the like, into tablets, granules, pills, capsules, powders, solutions, suspensions, injectable agents, liniment, and the like, by using standard techniques. The pharmaceutical preparations can be administered via oral route or percutaneous route, or via intravenous route.

The compound of the present invention can be administered to an adult patient in a dosage of 0.01 to 100 mg/kg, given as a single dose or in divided several doses per day. This dose can be appropriately increased or decreased depending on the type of diseases, age and body weight, symptoms of the patient, and the like.

The compounds of the present invention can be synthesized by, for example, the below-mentioned production method.

Scheme 1

[Ka 4]

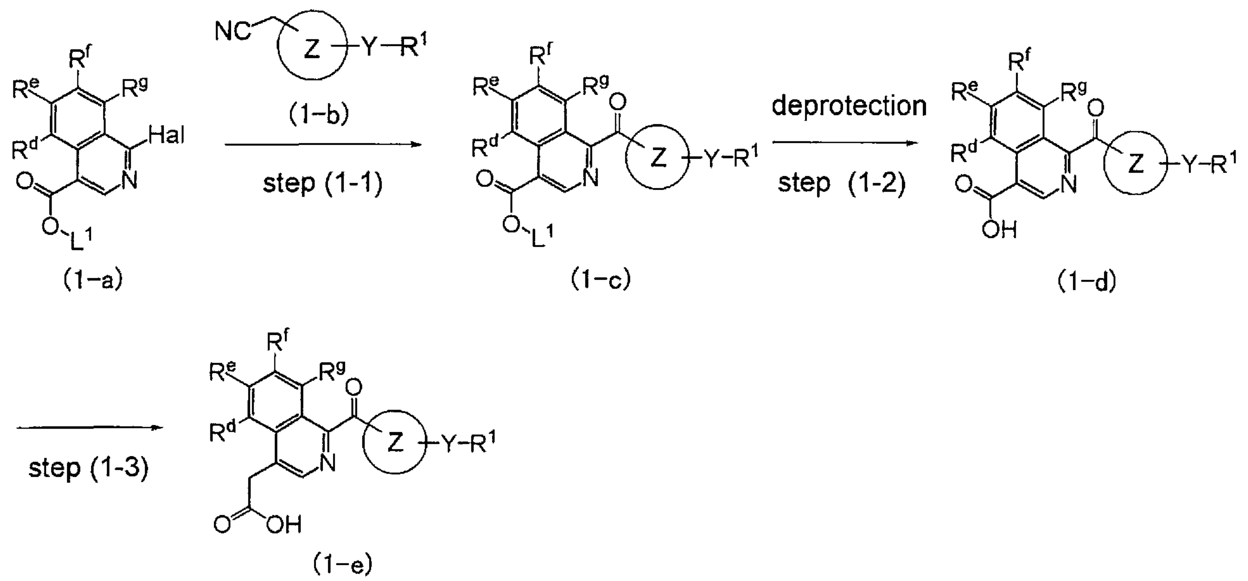


(1-a) (1-c) (1-d) (1-e)

In the scheme, Z, Y, $R^1$, $R^d$, $R^e$, $R^f$ and $R^g$ are the same as defined above, and Hal represents a chlorine atom, a bromine atom, and an iodine atom, and $L^1$ represents general protective groups of carboxylic acid, for example, groups described in Protective Groups in Organic Synthesis (third edition, 1999, P. G M. Wuts and T. Green) etc., and specifically represents a $C_{1-6}$ alkyl group, a benzyl group, a 4-methoxybenzyl group, or the like.

Step (1-1): Compound 1-c can be produced by allowing compound 1-a to react with compound 1-b in an ether solvent such as tetrahydrofuran and dioxane, a halogen solvent such as methylene chloride and chloroform, an aromatic hydrocarbon solvent such as toluene and xylene, or an aprotic polar solvent such as N,N-dimethylformamide, in the presence of bases such as sodium hydride, tert-butoxy potassium and sodium hexamethyldisilazide, and furthermore by stirring the reacted product in the presence of oxygen.

Step (1-2): This reaction may be carried out by the method described in, for example, Protective Groups in Organic Synthesis (third edition 1999, P. G M. Wuts and T. Green) etc., or methods similar to this method. Specifically, compound 1-d can be produced by subjecting compound 1-c to hydrolysis with mineral acid such as hydrochloric acid or an inorganic base such as sodium hydroxide and potassium hydroxide in an alcohol solvent such as methanol and ethanol, or in an ether solvent such as tetrahydrofuran and dioxane. When $L^1$ is a benzyl group or a 4-methoxybenzyl group, compound 1-d may be produced by subjecting compound 1-c to hydrogenation in an alcohol solvent such as methanol and ethanol, an ether solvent such as tetrahydrofuran and dioxane, a halogen solvent such as methylene chloride and chloroform, an aromatic hydrocarbon solvent such as toluene and xylene, in the presence of a catalyst such as palladium carbon. When $L^1$ is a 4-methoxybenzyl group, compound 1-d may be produced by deprotection reaction using ceric ammonium nitrate (CAN) or 2,3-dichloro-5,6-dicyano-1,4-benzoquinone (DDQ).

Step (1-3): Carboxylic acid chloride obtained by treating compound 1-d with oxalyl chloride, thionyl chloride, or the like, in a halogen solvent such as methylene chloride and chloroform or in an aromatic hydrocarbon solvent such as toluene and xylene, is treated with diazomethane, trimethylsilyl diazomethane, or the like, in ether solvent such as tetrahydrofuran and dioxane and in a polar solvent such as acetonitrile, or the like. Thereby, α-diazomethyl ketone can be produced. This compound is allowed to react with silver oxide, silver acetate, or the like, in a mixture solvent of water and an ether solvent such as tetrahydrofuran and dioxane, or in an aqueous solution, and thereby compound 1-e of the present invention can be produced.

Scheme 2

Scheme 2

[Ka 5]

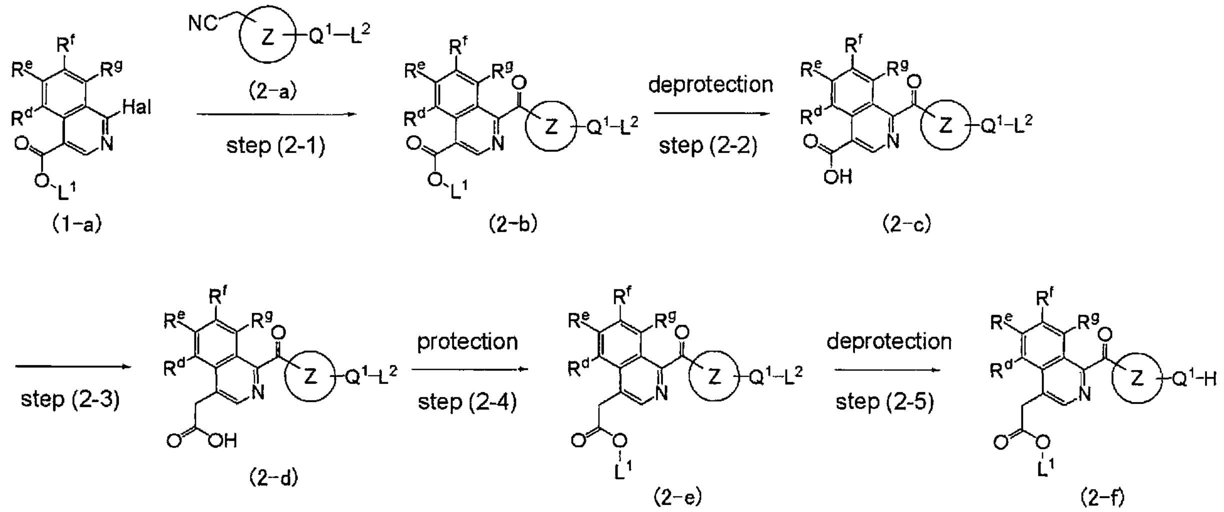


In the scheme, Z, $R^d$, $R^e$, $R^f$ $R^g$, $L^1$, and Hal are the same as defined above, and $Q^1$ represents the formula: -NH-, the formula: -O-, the formula: $-CO_2-$, the formula: $-CH_2O-$, the formula: $-CH_2NH-$, and $L^2$ represents general protective groups of aniline, phenol, carboxylic acid, primary amine, or primary alcohol, for example, groups described in Protective Groups in Organic Synthesis (third edition, 1999, P. G. M. Wuts and T. Green) etc., and specifically represents a tert-butoxycarbonyl group, a benzyl group, a 4-methoxybenzyl group, a methyl group, an acetyl group, a trimethylsilyl group, a tert-butyldimethylsilyl group, or the like, when $Q^1$ is the formula: -NH-, the formula: -O-, the formula: $-CH_2O-$, or the formula: $-CH_2NH-$, and represents a $C_{1-6}$ alkyl group, a benzyl group, a 4-methoxybenzyl group, or the like, when $Q^1$ is the formula: $-CO_2-$.

Step (2-1): Compound 2-b can be produced by using compound 2-a by the same procedure as used in step (1-1). Step (2-2): Compound 2-c can be produced by using compound 2-b by the same procedure as used in step (1-2). Step (2-3): Compound 2-d can be produced by using compound 2-c by the same procedure as used in step (1-3).

Step (2-4): Compound 2-e can be produced by subjecting compound 2-d to esterification with $C_{1-6}$ alkyl alcohol, benzyl alcohol, 4-methoxybenzyl alcohol, or the like in the presence of mineral acid such as sulfuric acid. Alternatively, compound 2-e may be produced by allowing compound 2-d to react with $C_{1-6}$ alkyl alcohol, benzyl alcohol, 4-methoxybenzyl alcohol, or the like, in ether solvent such as tetrahydrofuran and dioxane, a halogen solvent such as methylene chloride and chloroform, an aromatic hydrocarbon solvent such as toluene and xylene, or an aprotic polar solvent such as N,N-dimethylformamide, in the presence or the absence of a base such as triethylamine and pyridine, and in the presence of a condensing agent such as dicyclohexyl carbodiimide (DCC), 1-ethyl-3-(dimethylaminopropyl)-carbodiimide hydrochloride (EDC), benzotriazol-1-yloxytripyrrolidinophosphonium hexafluorophosphate (PyBOP (registered trademark)), 1-hydroxybenzotriazole hydrate (HOBt), or the like. Alternately, compound 2-e may be produced by reacting carboxylic acid chloride obtained by treating compound 2-d with oxalyl chloride, thionyl chloride, or the like, in a halogen solvent such as methylene chloride and chloroform or an aromatic hydrocarbon solvent such as toluene and xylene, with $C_{1-6}$ alkyl alcohol, benzyl alcohol, 4-methoxybenzyl alcohol, or the like. Furthermore, when $L^1$ is a methyl group, compound 2-e may be produced by allowing compound 2-d to react with diazomethane, trimethylsilyl diazomethane, or the like, in an alcohol solvent such as methanol and ethanol. Also, compound 2-e may be produced by allowing compound 2-d to react with iodomethane in an ether solvent such as tetrahydrofuran and dioxane, a halogen solvent such as methylene chloride and chloroform, an aromatic hydrocarbon solvent such as toluene and xylene, or an aprotic polar solvent such as N,N-dimethylformamide in the presence of a base such as triethylamine, pyridine, potassium carbonate, or the like.

Step (2-5): This reaction may be carried out by the method described in, for example, Protective Groups in Organic Synthesis (third edition 1999, P G M. Wuts and T. Green) etc., or methods similar to this method. Specifically, when $L^2$ is a tert- butoxycarbonyl group, a tert-butyl group, a 4-methoxybenzyl group, or a trimethylsilyl group, compound 2-f can be produced by subjecting compound 2-e to deprotection reaction using mineral acid such as hydrochloric acid, acetic acid, trifluoroacetic acid, or the like, in an ether solvent such as tetrahydrofuran and dioxane, a halogen solvent such as methylene chloride and chloroform, or an aromatic hydrocarbon solvent such as toluene and xylene. When $L^2$

is a benzyl group or a 4-methoxybenzyl group, compound 2-f may be produced by subjecting compound 2-e to hydrogenation in an alcohol solvent such as methanol and ethanol, an ether solvent such as tetrahydrofuran and dioxane, a halogen solvent such as methylene chloride and chloroform, an aromatic hydrocarbon solvent such as toluene and xylene, in the presence of a catalyst such as palladium carbon. When $L^2$ is a trimethylsilyl group or a tert- butyldimethylsilyl group, compound 2-f can be produced by treating compound 2-e with potassium fluoride, tetrabutylammonium fluoride, or the like. When $Q^1$ is the formula: -O- or the formula: $-CH_2O-$, and $L^2$ is a methyl group, compound 2-f can be produced by treating compound 2-e with $BBr_3$ in a halogen solvent such as methylene chloride and chloroform or an aromatic hydrocarbon solvent such as toluene and xylene. When $L^2$ is an acetyl group, compound 2-f can be produced by subjecting compound 2-e to hydrolysis with mineral acid such as hydrochloric acid or an inorganic base such as sodium hydroxide and potassium hydroxide in an alcohol solvent such as methanol and ethanol or an ether solvent such as tetrahydrofuran and dioxane. When $Q^1$ is the formula:$-CO_2-$, compound 2-f can be produced by the same procedure as used in step (1-2).

Scheme 3

[Ka 6]

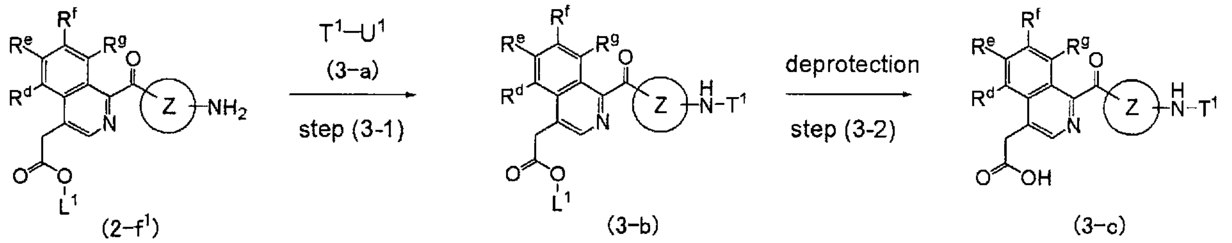


(2-$f^1$) (3-b) (3-c)

In the scheme, Z, $R^d$, $R^e$, $R^f$ $R^g$, and $L^1$ are the same as defined above, and $T^1$ represents the formula: $-CO-W-R^1$, the formula: $CO_2-W-R^1$, the formula: $-CO-W-O-R^1$, the formula: $-SO_2-W-R^1$, or the formula: $-CO-W-NR^4SO_2-R^1$ (W, $R^1$, and $R^4$ are the same as defined above), $U^1$ represents a general leaving group, for example, a chlorine atom, a bromine atom, an iodine atom, a phenoxy group, an imidazolyl group, a triazolyl group, and the like.

Step (3-1): When $U^1$ is a chlorine atom, a bromine atom, an iodine atom, a phenoxy group, an imidazolyl group, or a triazolyl group, compound 3-b can be produced by allowing compound 3-a to react with compound 2-$f^1$ in an ether solvent such as tetrahydrofuran and dioxane, a halogen solvent such as methylene chloride and chloroform, an aromatic hydrocarbon solvent such as toluene and xylene, or an aprotic polar solvent such as N,N-dimethylformamide, in the presence or absence of a base such as triethylamine and pyridine. Also, compound 3-b may be produced by allowing compound 3-a to react with compound 2-$f^1$ by using a base such as pyridine and triethylamine as a solvent. When $T^1$ is the formula: $-COW-R^1$, the formula: $-CO-W-O-R^1$, or the formula: $-CO-W-NR^4SO_2-R^1$, $U^1$ may be a hydroxyl group, and compound 3-b may be produced by allowing compound 3-a with compound 2-$f^1$ in an ether solvent such as tetrahydrofuran and dioxane, a halogen solvent such as methylene chloride and chloroform, an aromatic hydrocarbon solvent such as toluene and xylene, or an aprotic polar solvent such as N,N-dimethylformamide, in the presence or absence of a base such as triethylamine and pyridine, and in the presence of a condensing agent such as dicyclohexyl carbodiimide (DCC), I-ethyl-3-(dimethylaminopropyl)-carbodiimide hydrochloride (EDC), benzotriazol-1-yloxytripyrrolidinophosphonium hexafluorophosphate (PyBOP (registered trademark)), 1-hydroxybenzotriazole hydrate (HOBt), or the like.

Step (3-2): Compound 3-c can be produced by using compound 3-b by the same procedure as used in step (1-2).

Scheme 4

[Ka 7]

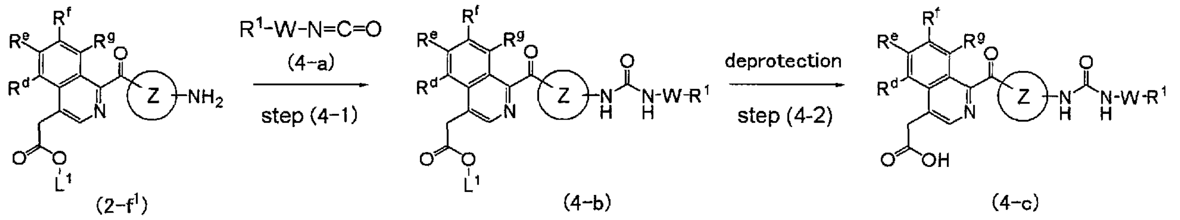


(2-$f^1$) (4-b) (4-c)

In the scheme, Z, $R^1$, $R^d$, $R^e$, $R^f$ $R^g$, W, and $L^1$ are the same as defined above.
Step (4-1): Compound 4-b can be produced by allowing compound 4-a to react with compound 2-$f^1$ in an ether solvent such as tetrahydrofuran and dioxane, a halogen solvent such as methylene chloride and chloroform, an aromatic hydrocarbon solvent such as toluene and xylene, or an aprotic polar solvent such as N,N-dimethylformamide.

Step (4-2): Compound 4-c can be produced by using compound 4-b by the same procedure as used in step (1-2).

Scheme 5

[Ka 8]

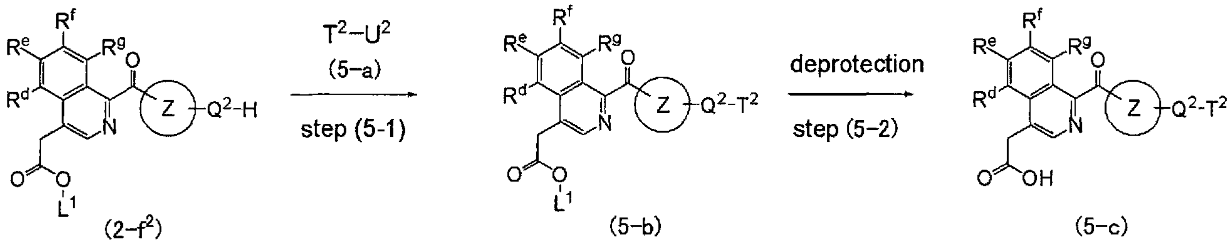


(2–$f^2$) (5–b) (5–c)

In the scheme, Z, $R^d$, $R^e$, $R^f$, $R^g$ and $L^1$ are the same as defined above, , and $Q^2$ represents the formula: -NH-, the formula: -O-, the formula: $-CH_2O-$, or the formula: $-CH_2NH-$, $T^2$ represents the formula: -W-$R^1$, or the formula: -W-O-$R^1$ (W, $R^1$ are the same as defined above), $U^2$ represents a general leaving group, for example, a chlorine atom, a bromine atom, an iodine atom, a methane sulfonyloxy group, a p-toluene sulfonyloxy group, or the like.

Step (5-1): Compound 5-b can be produced by allowing compound 5-a to react with compound 2-$f^2$ in an ether solvent such as tetrahydrofuran and dioxane, a halogen solvent such as methylene chloride and chloroform, an aromatic hydrocarbon solvent such as toluene and xylene, or an aprotic polar solvent such as N,N-dimethylformamide, in the presence or absence of a base such as triethylamine, pyridine, and potassium carbonate. When $Q^2$ is the formula: -O-, $U^2$ may be a hydroxyl group, and compound 5-b may be produced by allowing compound 5-a to react with compound 2-$f^2$ in an ether solvent such as tetrahydrofuran and dioxane, a halogen solvent such as methylene chloride and chloroform, an aromatic hydrocarbon solvent such as toluene and xylene, or an aprotic polar solvent such as N,N-dimethylformamide, in the presence of a reagent such as triphenylphosphine and tri-n-butyl phosphine, diethyl azodicarboxylate and tetramethyl azodicarboxy amide.

Step (5-2): Compound 5-c can be produced by using compound 5-b by the same procedure as used in step (1-2).

Scheme 6

[Ka 9]

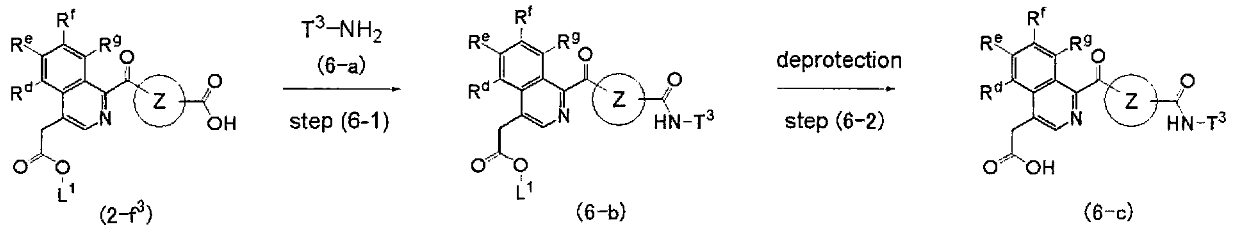

(2-$f^3$) (6-b) (6-c)

In the scheme, Z, $R^d$, $R^e$, $R^f$ $R^g$ and $L^1$ are the same as defined above, and $T^3$ represents the formula: -W-$R^1$, the formula: -W-O-$R^1$, or the formula: -W-$NR^4CO$-$R^1$ (W, $R^1$ and $R^4$ are the same as defined above).

Step (6-1): Compound 6-b can be produced by allowing compound 6-a to react with compound 2-$f^3$ in an ether solvent such as tetrahydrofuran and dioxane, a halogen solvent such as methylene chloride and chloroform, an aromatic hydrocarbon solvent such as toluene and xylene, or an aprotic polar solvent such as N,N-dimethylformamide, in the presence or absence of a base such as triethylamine and pyridine, in the presence of a condensing agent such as dicyclohexyl carbodiimide (DCC), 1-ethyl-3-(dimethylaminopropyl)-carbodiimide hydrochloride (EDC), benzotriazol-1-yloxytripyrrolidinophosphonium hexafluorophosphate (PyBOP (registered trademark)), 1-hydroxybenzotriazole hydrate (HOBt), or the like. Alternatively, compound 6-b may be produced by allowing carboxylic acid chloride obtained by treating compound 2-$f^3$ with oxalyl chloride, thionyl chloride, or the like, in a halogen solvent such as methylene chloride and chloroform, or an aromatic hydrocarbon solvent such as toluene and xylene to react with compound 6-a in an ether solvent such as tetrahydrofuran and dioxane, a halogen solvent such as methylene chloride and chloroform, or an aromatic hydrocarbon solvent such as toluene and xylene.

Step (6-2): Compound 6-c can be produced by using compound 6-b by the same procedure as used in step (1-2).

Scheme 7

[Ka 10]

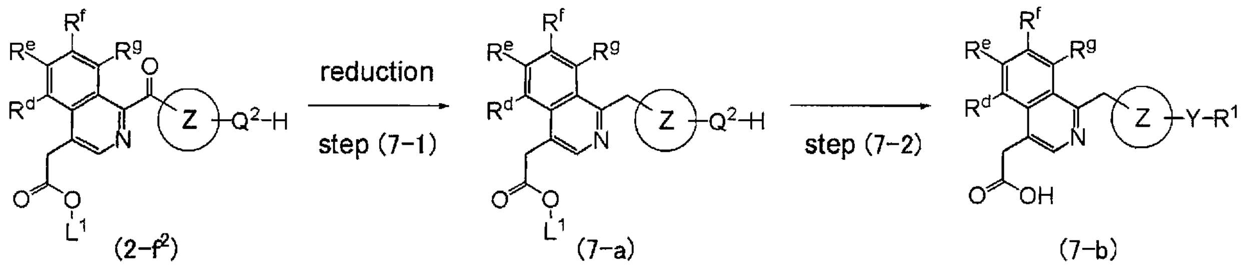

(2-$f^2$) (7-a) (7-b)

In the scheme, Z, $R^1$, $R^d$, $R^e$, $R^f$ $R^g$, $L^1$, and $Q^2$ are the same as defined above.

Step (7-1): Compound 7-a can be produced by treating compound 2-$f^2$ with a reducing agent such as sodium borohydride and lithium aluminum hydride in an alcohol solvent such as methanol and ethanol, an ether solvent such as tetrahydrofuran and dioxane, a halogen solvent such as methylene chloride and chloroform, or an aromatic hydrocarbon solvent such as toluene and xylene. Compound 7-a may be produced by reacting with trimethylsilane in an ether solvent such as tetrahydrofuran and dioxane, a halogen solvent such as methylene chloride and chloroform, or an aromatic hydrocarbon solvent such as toluene and xylene in the presence of trifluoroacetic acid, boron trifluoride etherate, or the like. Alternatively, compound 7-a may be produced by hydrogenating compound 2-$f^2$ in an alcohol solvent such as methanol and ethanol, an ether solvent such as tetrahydrofuran and dioxane, a halogen solvent such as methylene chloride and

chloroform, or an aromatic hydrocarbon solvent such as toluene and xylene, in the presence of a catalyst such as palladium carbon.

Step (7-2): Compound 7-b can be produced by using compound 7-a by the same procedure as in the schemes 3 to 5. In this case, however, Y represents the formula: $-NR^3COW-$, the formula: $-NR^3CO-W-O-$, the formula: $-NR^3CO_2-W-$, the formula: $-NR^3-W-$, the formula: $-NR^3SO_2-W-$, the formula: $-NR^3CONR^4-W-$, the formula: $-NR^3CO-W-NR^4SO_2-$, the formula: - $CH_2-O-W-$, the formula: $-CH_2NR^3-W-$, the formula: -O-W-, or the formula: -O-W-O- (W, $R^3$, and $R^4$ are the same as defined above).

Scheme 8

[Ka 11]

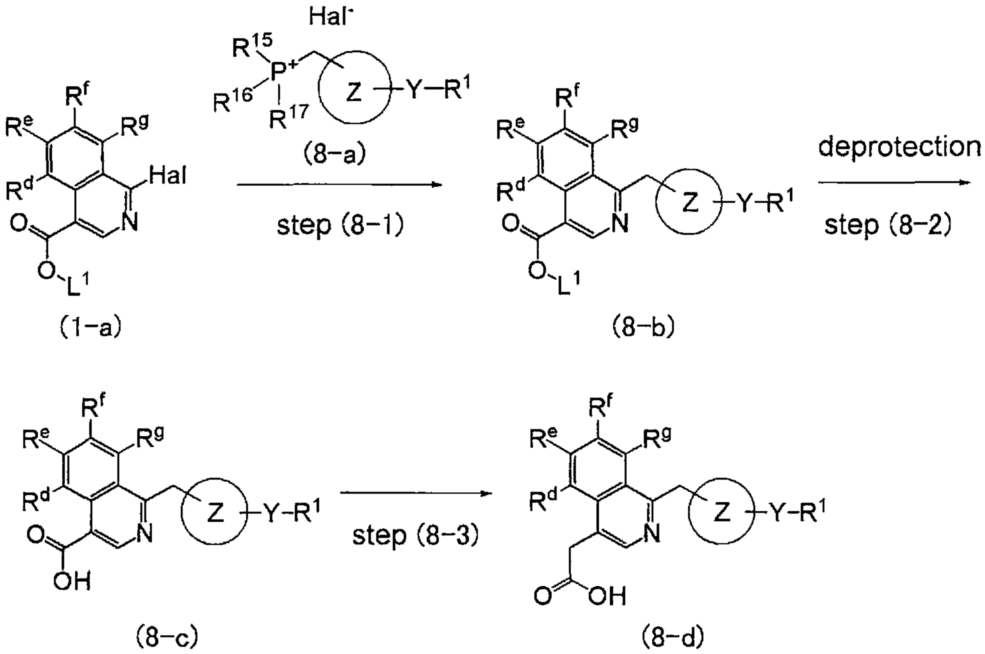


In the scheme, Z, Y, $R^1$, $R^d$, $R^e$, $R^f$ $R^g$, $L^1$, and Hal are the same as defined above, and $R^{15}$, $R^{16}$, and $R^{17}$ represent a $C_{1-6}$ alkyl group.

Step (8-1): Compound 8-b can be produced by allowing compound 8-a to react with compound 1-a in an ether solvent such as tetrahydrofuran and dioxane, a halogen solvent such as methylene chloride and chloroform, an aromatic hydrocarbon solvent such as toluene and xylene, or an aprotic polar solvent such as N,N-dimethylformamide, in the presence of a base such as sodium hydride, tert-butoxy potassium and sodium hexamethyldisilazide, and further treating the reacted product in an aqueous solution of sodium carbonate, potassium carbonate, or the like.

Step (8-2): Compound 8-c can be produced by using compound 8-b by the same procedure as used in step (1-2). Step (8-3): Compound 8-d can be produced by using compound 8-c by the same procedure as used in step (1-3).

Scheme 9

[Ka 12]

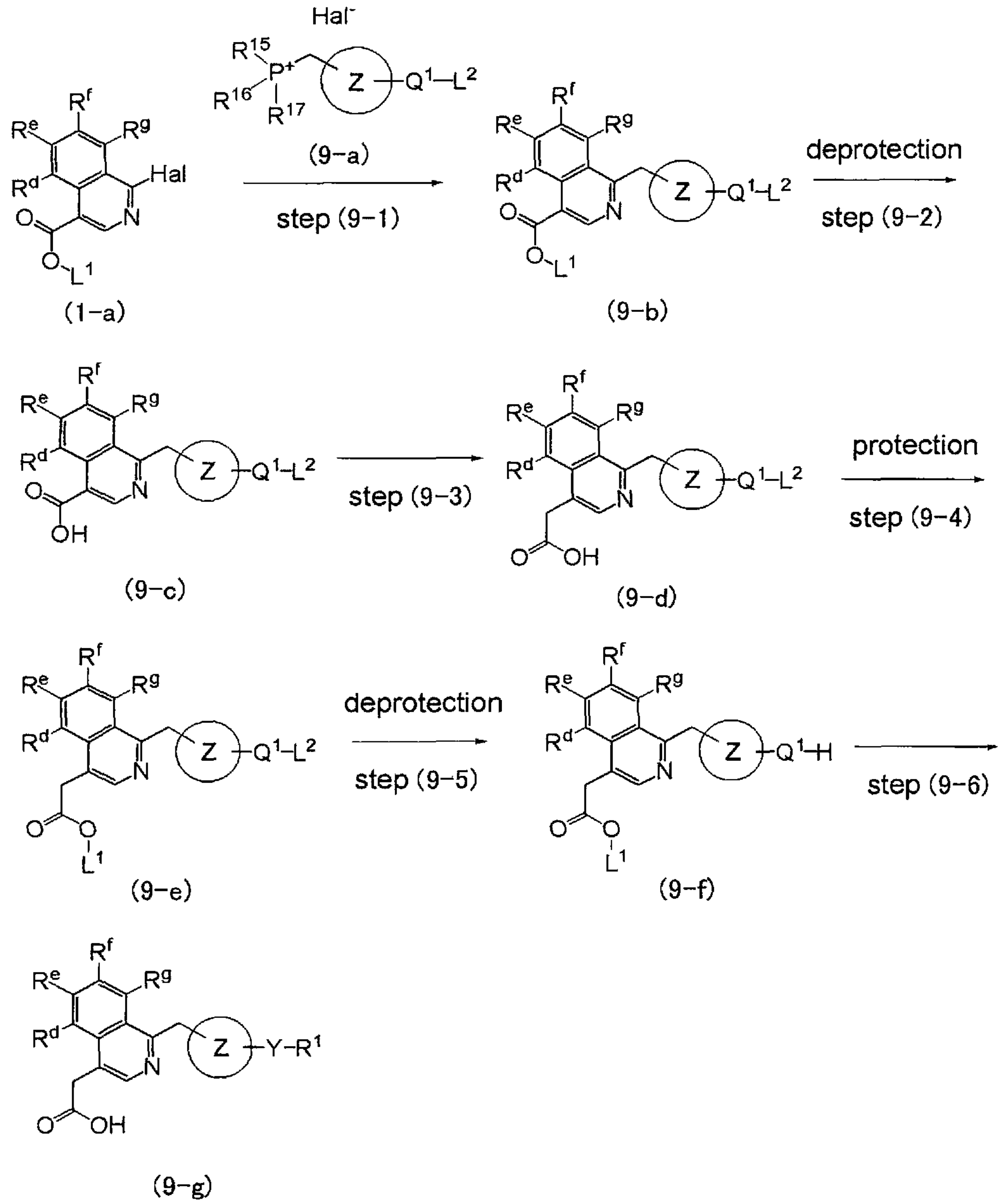


In the scheme, Z, $R^1$, $R^d$, $R^e$, $R^f$ $R^g$, $R^{15}$, $R^{16}$, $R^{17}$, $Q^1$, $L^1$, $L^2$, and Hal are the same as defined above.
Step (9-1): Compound 9-b can be produced by using compound 9-a by the same procedure as used in step (8-1).
Step (9-2): Compound 9-c can be produced by using compound 9-b by the same procedure as used in step (1-2).
Step (9-3): Compound 9-d can be produced by using compound 9-c by the same procedure as used in step (1-3).
Step (9-4): Compound 9-e can be produced by using compound 9-d by the same procedure as used in step (2-4).
Step (9-5): Compound 9-f can be produced by using compound 9-e by the same procedure as used in step (2-5).

Step (9-6): Compound 9-g can be produced by using compound 9-fby the same procedure as in the schemes 3 to 6. In this case, however, Y represents the formula: $-NR^3COW-$, the formula: $-NR^3CO-W-O-$, the formula: $-NR^3CO_2-W-$, the formula: $-NR^3-W-$, the formula: $-NR^3SO_2-W-$, the formula: $-NR^3CONR^4-W-$, the formula: $-NR^3CO-W-NR^4SO_2-$, the formula: - $CONR^3-W-$, the formula: $-CONR^3-W-O-$, the formula: $-CH_2-O-W-$, the formula: $-CH_2NR^3-W-$, the formula: $-CONR^3-W-NR^4CO-$, the formula: -O-W-, or the formula: -O-W-O- (W, $R^3$, and $R^4$ are the same as defined above).

Scheme 10

[Ka 13]

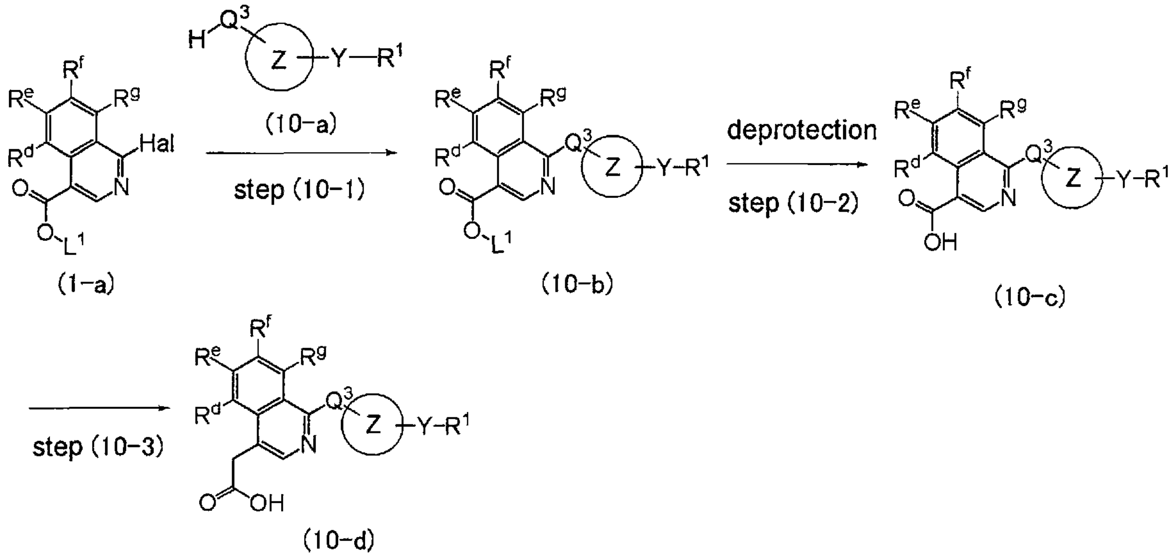


(1-a) (10-a) (10-b) (10-c) (10-d)

In the scheme, Z, Y, $R^1$, $R^d$, $R^e$, $R^f$ $R^g$, $L^1$, and Hal are the same as defined above, and $Q^3$ represents an oxygen atom, a sulfur atom, or the formula: $-NR^2-$ ($R^2$ is the same as defined above).

Step (10-1): Compound 10-b can be produced by allowing compound 10-a to react with compound 1-a in an ether solvent such as tetrahydrofuran and dioxane, a halogen solvent such as methylene chloride and chloroform, an aromatic hydrocarbon solvent such as toluene and xylene, or an aprotic polar solvent such as N,N-dimethylformamide, in the presence or absence of a base such as triethylamine, pyridine, and potassium carbonate.

Step (10-2): Compound 10-c can be produced by using compound 10-b by the same procedure as used in step (1-2).

Step (10-3): Compound 10-d can be produced by using compound 10-c by the same procedure as used in step (1-3).

Scheme 11

[Ka 14]

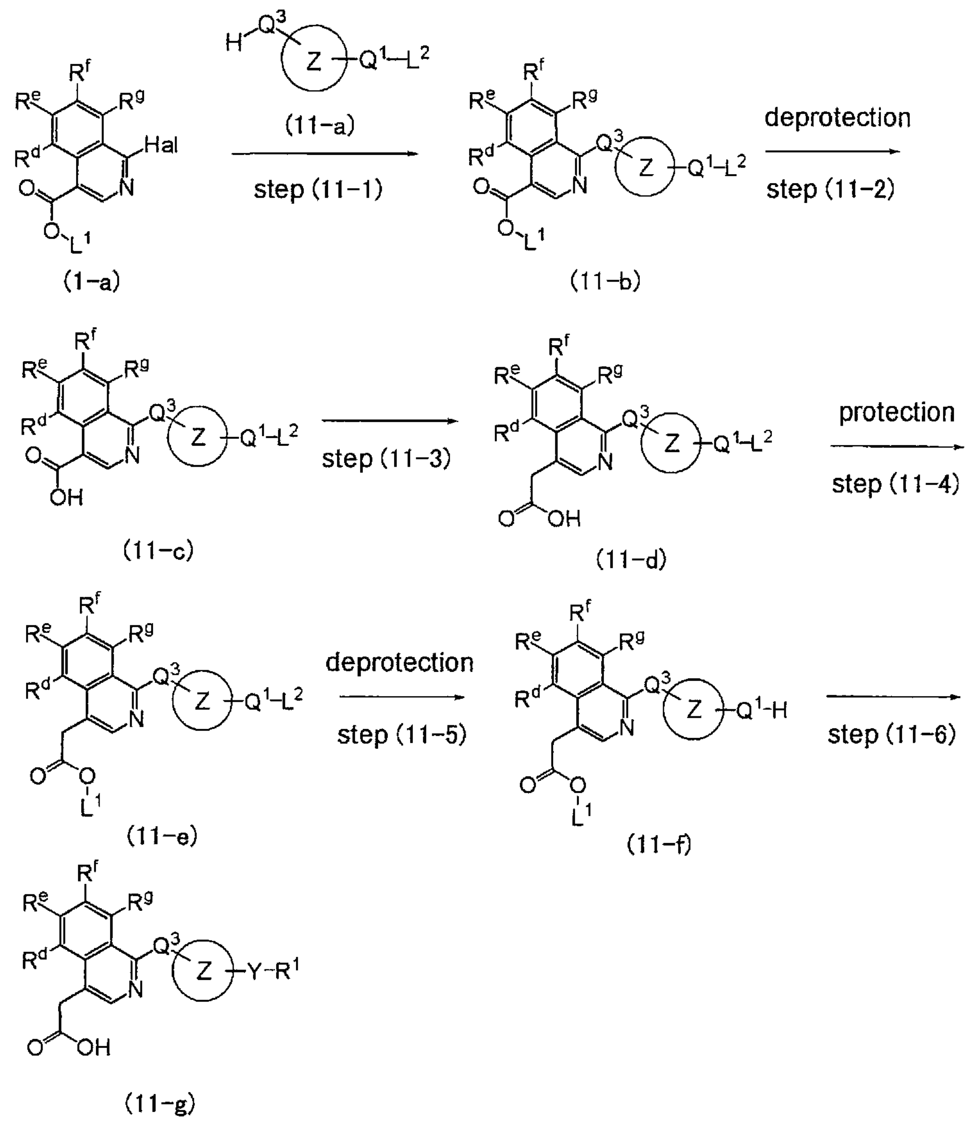


(1-a) (11-b) (11-c) (11-d) (11-e) (11-f) (11-g)

In the scheme, Z, Y, $R^1$, $R^d$, $R^e$, $R^f$, $R^g$, $Q^1$, $Q^3$, $L^1$, $L^2$, and Hal are the same as defined above.
Step (11-1): Compound 11-b can be produced by using compound 11-a by the same procedure as used in step (10-1).
Step (11-2): Compound 11-c can be produced by using compound 11-b by the same procedure as used in step (1-2).
Step (11-3): Compound 11-d can be produced by using compound 11-c by the same procedure as used in step (1-3).
Step (11-4): Compound 11-e can be produced by using compound 11-d by the same procedure as used in step (2-4).
Step (11-5): Compound 11-f can be produced by using compound 11-e by the same procedure as used in step (2-5).

Step (11-6): Compound 11-g can be produced by using compound 11-f by the same procedure as in the schemes 3 to 6. In this case, however, Y represents the formula: - $NR^3CO$-W-, the formula: -$NR^3CO$-W-O-, the formula: -$NR^3CO_2$-W-, the formula: -$NR^3$-W-, the formula: -$NR^3SO_2$-W-, the formula: -$NR^3CONR^4$-W-, the formula: -$NR^3CO$-W-$NR^4SO_2$-, the formula: -$CONR^3$-W-, the formula: -$CONR^3$-W-O-, the formula: -$CH_2$-O-W-, the formula: - $CH_2NR^3$-W-, the formula: -$CONR^3$-W-$NR^4CO$-, the formula: -O-W-, or the formula: -O-W-O-(W, $R^3$, and $R^4$ are the same as defined above).

Scheme 12

[Ka 15]

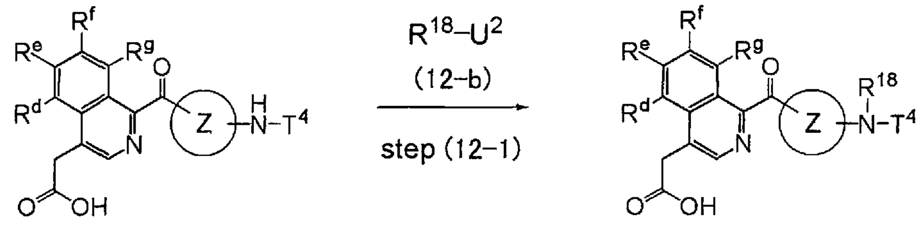


(12-a) (12-c)

In the scheme, Z, $R^d$, $R^e$, $R^f$ $R^g$, and $U^2$ are the same as defined above; $T^4$ represents the formula: -W-$R^1$, the formula: -CO-W-$R^1$, the formula: -$CO_2$-W-$R^1$, the formula: - CO-W-O-$R^1$, or the formula: -$SO_2$-W-$R^1$ (W, and $R^1$ are the same as defined above); and $R^{18}$ is a $C_{1-6}$ alkyl group.

Step (12-1): Compound 12-c of the present invention can be produced by allowing compound 12-b to react with compound 12-a in an ether solvent such as tetrahydrofuran and dioxane, a halogen solvent such as methylene chloride and chloroform, an aromatic hydrocarbon solvent such as toluene and xylene, and an aprotic polar solvent such as N,N-dimethylformamide, in the presence of a base such as sodium hydride. Furthermore, a compound of the present invention in which a nitrogen atom is $C_{1-6}$ alkylated can be produced by carrying out the same reaction using compound 4-c, compound 6-c, compound 5-c in which $Q^2$ is the formula: -$CH^2$NH-.

Scheme 13

[Ka 16]

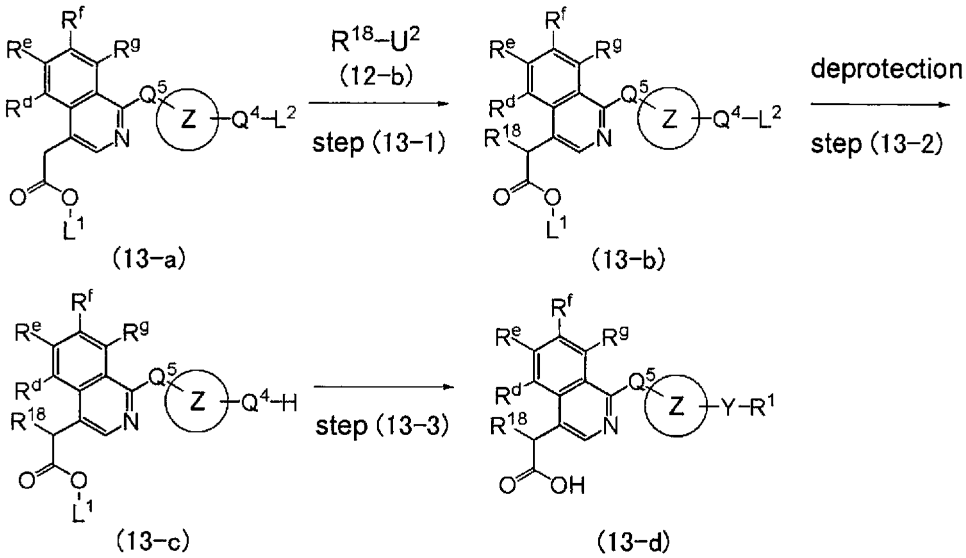


(13-a) (13-b) (13-c) (13-d)

In the scheme, Z, Y, $R^1$, $R^d$, $R^e$, $R^f$ $R^g$, $R^{18}$, $L^1$, $L^2$, and $U^2$ are the same as defined above, $Q^4$ represents the formula: -O-, the formula: -$CO_2$-, or the formula: -$CH_2O$-; and $Q^5$ represents the formula: -$CR^hR^i$-, the formula -CO-, or formula (II).

Step (13-1): Compound 13-b can be produced by allowing compound 12-b to react with compound 13-a in an ether solvent such as tetrahydrofuran and dioxane, a halogen solvent such as methylene chloride and chloroform, an

aromatic hydrocarbon solvent such as toluene and xylene, and an aprotic polar solvent such as N,N-dimethylformamide, in the presence of a base such as sodium hydride and tert-butoxy potassium.

Step (13-2): Compound 13-c can be produced by using compound 13-b by the same procedure as used in step (2-5).

Step (3-3): A compound of the present invention 13-d can be produced by using compound 13-c by the same procedure as in the schemes 5 to 6. In this case, however, Y represents the formula: -O-W-, the formula: -O-W-O-, the formula: $-CH_2-O-W-$, the formula: - $CONR^3$-W-, the formula: $-CONR^3$-W-O-, or the formula: $-CONR^3-W-NR^4CO-$ (W, $R^3$, and $R^4$ are the same as defined above).

Scheme 14

[Ka 17]

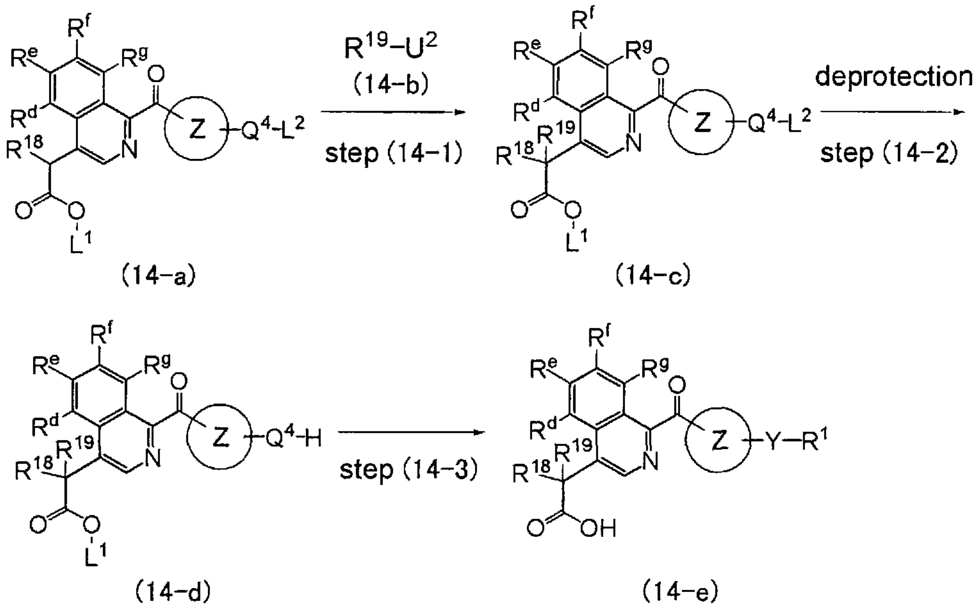


(14-a) (14-c) (14-d) (14-e)

In the scheme, Z, Y, $R^1$, $R^d$, $R^e$, $R^f$ $R^g$, $R^{18}$, $L^1$, $L^2$, $U^2$ and $Q^4$ are the same as defined above, and $R^{19}$ represents a $C_{1-6}$ alkyl group.

Step (14-1): Compound 14-c can be produced by using compound 14-b by the same procedure as used in step (13-1).

Step (14-2): Compound 14-d can be produced by using compound 14-b by the same procedure as used in step (2-5).

Step (14-3): Compound 14-e can be produced by using compound 14-d by the same procedure as in the schemes 5 to 6. In this case, however, Y represents the formula: -O-W-, the formula: -O-W-O-, the formula: $-CH_2-O-W-$, the formula: $-CONR^3$-W-, the formula: - $CONR^3$-W-O-, or the formula: $-CONR^3-W-NR^4CO-$ (W, $R^3$, and $R^4$ are the same as defined above).

Scheme 15

[Ka 18]

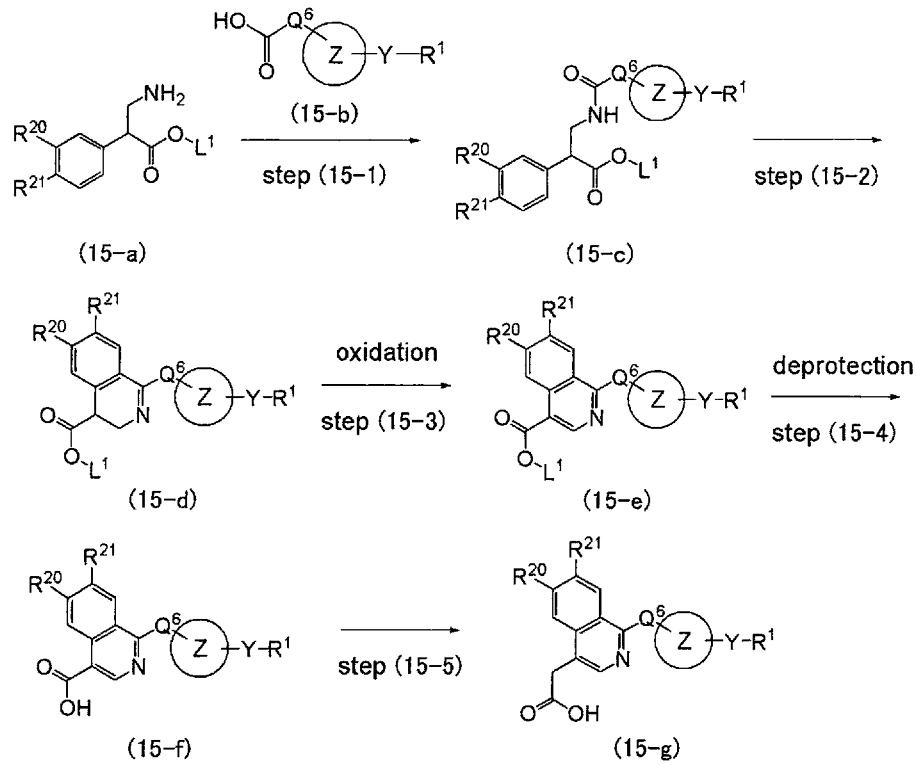

(15-a) (15-c) (15-d) (15-e) (15-f) (15-g)

In the scheme, Z, Y, $R^1$, and $L^1$ are the same as defined above; $R^{20}$ and $R^{21}$ represent a $C_{1-6}$ alkoxy group or a hydrogen atom; and $Q^6$ represents the formula: $-CR^hR^i-$, or formula (II).

Step (15-1): Compound 15-c can be produced by allowing compound 15-b to react with compound 15-a in an ether solvent such as tetrahydrofuran and dioxane, a halogen solvent such as methylene chloride and chloroform, an aromatic hydrocarbon solvent such as toluene and xylene, or an aprotic polar solvent such as N,N-dimethylformamide, in the presence or absence of a base such as triethylamine and pyridine, and in the presence of a condensing agent such as dicyclohexyl carbodiimide (DCC), 1-ethyl-3-(dimethylaminopropyl)-carbodiimide hydrochloride (EDC), benzotriazol-1-yloxytripyrrolidinophosphonium hexafluorophosphate (PyBOP (registered trademark)), 1-hydroxybenzotriazole hydrate (HOBt), or the like. Alternatively, compound 15-c may be produced by allowing a carboxylic acid chloride obtained by treating compound 15-b with oxalyl chloride, thionyl chloride, or the like, in a halogen solvent such as methylene chloride and chloroform, or an aromatic hydrocarbon solvent such as toluene and xylene with compound 15-a in an ether solvent such as tetrahydrofuran and dioxane, a halogen solvent such as methylene chloride and chloroform, and an aromatic hydrocarbon solvent such as toluene and xylene.

Step (15-2): Compound 15-d can be produced by treating compound 15-c with phosphorus oxychloride, diphosphorus pentaoxide, polyphosphoric acid, trifluoroacetic anhydride, trifluoromethanesulfonic anhydride, or the like, in a halogen solvent such as methylene chloride and chloroform, or an aromatic hydrocarbon solvent such as toluene and xylene, in the presence or absence of additives such as 2-chloropyridine.

Step (15-3): Compound 15-e can be produced by treating compound 15-d with sulfur. Alternatively, compound 15-e may be produced by treating compound 15-d with palladium carbon or the like in an aromatic hydrocarbon solvent such as toluene and xylene, or an aliphatic hydrocarbon solvent such as decahydronaphthalene.

Step (15-4): Compound 15-f can be produced by using compound 15-e by the same procedure as used in step (1-2).

Step (15-5): Compound 15-g can be produced by using compound 15-f by the same procedure as used in step (1-3).

Scheme 16

[Ka 19]

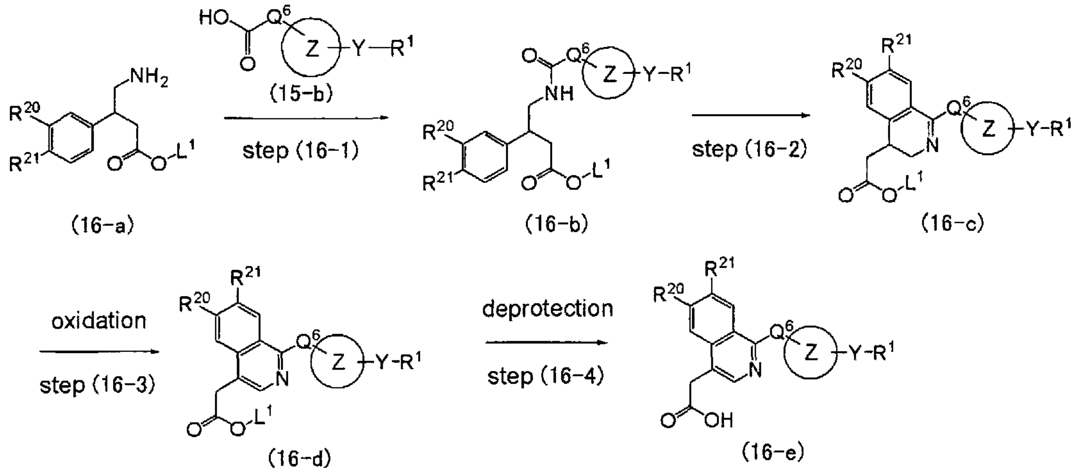


In the scheme, Z, Y, $R^1$, $R^{20}$, $R^{21}$, $L^1$, and $Q^6$ are the same as defined above.
Step (16-1): Compound 16-b can be produced by using compound 16-a by the same procedure as used in step (15-1).
Step (16-2): Compound 16-c can be produced by using compound 16-b by the same procedure as used in step (15-2).
Step (16-3): Compound 16-d can be produced by using compound 16-c by the same procedure as used in step (15-3).
Step (16-4): Compound 16-e can be produced by using compound 16-d by the same procedure as used in step (1-2).

Scheme 17

[Ka 20]

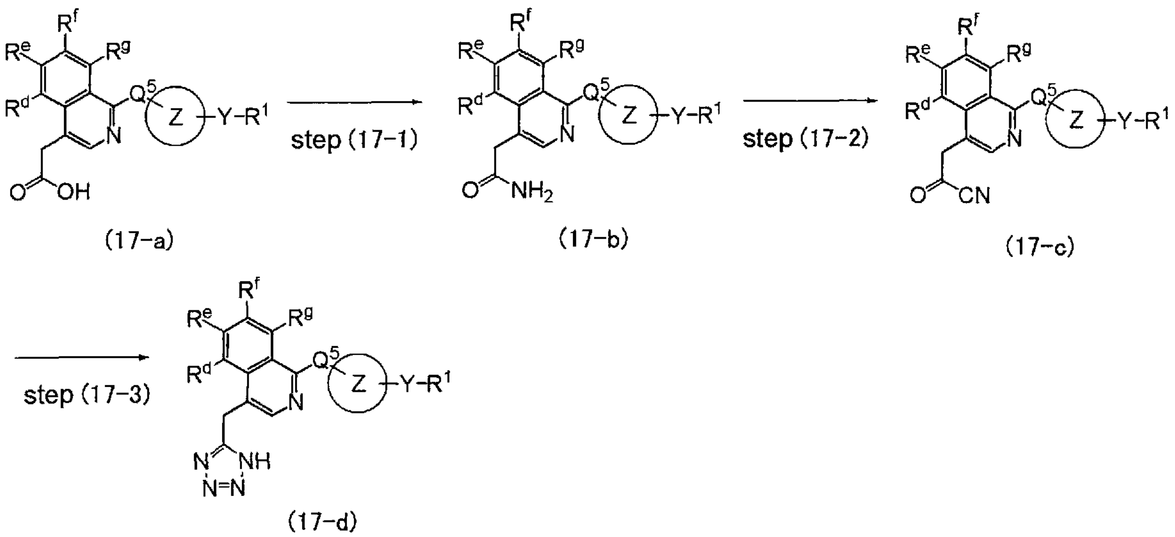


In the scheme, Z, Y, $R^1$, $R^d$, $R^e$, $R^f$ $R^g$, and $Q^5$ are the same as defined above.
Step (17-1): Compound 17-b can be produced by allowing compound 17-a with aqueous ammonia solution in an ether solvent such as tetrahydrofuran and dioxane, a halogen solvent such as methylene chloride and chloroform, an aromatic hydrocarbon solvent such as toluene and xylene, or an aprotic polar solvent such as N,N-dimethylformamide, in the

presence or absence of a base such as triethylamine and pyridine, and in the presence of a condensing agent such as dicyclohexyl carbodiimide (DCC), 1-ethyl-3-(dimethylaminopropyl)-carbodiimide hydrochloride (EDC), benzotriazol-1-yloxytripyrrolidinophosphonium hexafluorophosphate (PyBOP (registered trademark)), 1-hydroxybenzotriazole hydrate (HOBt), and 1,1'-carbonyl diimidazole (CDI). Alternatively, compound 17-b of the present invention may be produced by allowing a carboxylic acid chloride obtained by treating compound 17-a with oxalyl chloride, thionyl chloride, or the like, in a halogen solvent such as methylene chloride and chloroform, or an aromatic hydrocarbon solvent such as toluene and xylene with an aqueous ammonia solution in an ether solvent such as tetrahydrofuran and dioxane, a halogen solvent such as methylene chloride and chloroform, and an aromatic hydrocarbon solvent such as toluene and xylene.

Step (17-2): Compound 17-c can be obtained by treating compound 17-b with phosphoryl chloride, thionyl chloride, oxalyl chloride, or the like, in a halogen solvent such as methylene chloride and chloroform, or an aromatic hydrocarbon solvent such as toluene and xylene.

Step (17-3): A compound of the present invention 17-d can be produced by allowing compound 17-c to react with sodium azide in an ether solvent such as tetrahydrofuran and dioxane, a halogen solvent such as methylene chloride and chloroform, an aromatic hydrocarbon solvent such as toluene and xylene, or an aprotic polar solvent such as N,N-dimethylformamide, in the presence or absence of triethylamine hydrochloride, ammonium chloride, or the like.

Scheme 18

[Ka 21]

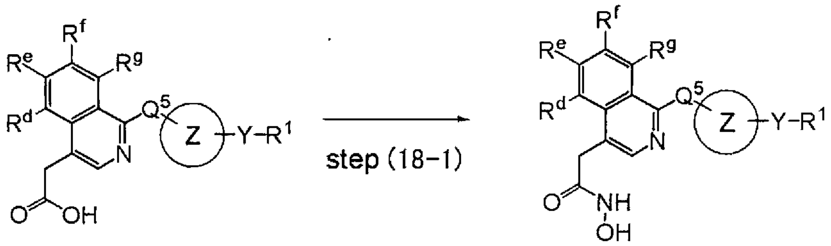


(17-a) (18-a)

In the scheme, Z, Y, $R^1$, $R^d$, $R^e$, $R^f$, $R^g$, and $Q^5$ are the same as defined above.

Step (18-1): Compound 18-a of the present invention can be produced by allowing compound 17-a with hydroxylamine or hydroxylamine hydrochloride in ether solvent such as tetrahydrofuran and dioxane, a halogen solvent such as methylene chloride and chloroform, an aromatic hydrocarbon solvent such as toluene and xylene, or an aprotic polar solvent such as N,N-dimethylformamide, in the presence or the absence of a base such as triethylamine and pyridine, and in the presence of a condensing agent such as dicyclohexyl carbodiimide (DCC), 1-ethyl-3-(dimethylaminopropyl)-carbodiimide hydrochloride (EDC), benzotriazol-1-yloxytripyrrolidinophosphonium hexafluorophosphate (PyBOP (registered trademark)), 1-hydroxybenzotriazole hydrate (HOBt), 1,1'-carbonyl diimidazole (CDI). Compound 18-a of the present invention can be produced by the same procedure by using a reagent such as O-(tetrahydro-2H-pyran-2-yl)hydroxylamine, and O-benzyl hydroxylamine in which a hydroxyl group is protected, instead of using hydroxylamine. Then, the obtained compound is subjected to deprotection reaction by, for example, a method described in Protective Groups in Organic Synthesis (third edition 1999, P. G M. Wuts and T. Green) etc., or methods similar to this method, and thus compound 18-a of the present invention can be produced. Specifically, when the protecting group is a tetrahydropyranyl group, compound 18-a of the present invention can be produced by deprotection reaction using mineral acid such as hydrochloric acid, acetic acid, trifluoroacetic acid, or the like, in an ether solvent such as tetrahydrofuran and dioxane, a halogen solvent such as methylene chloride and chloroform, or an aromatic hydrocarbon solvent such as toluene and xylene. When the protecting group is a benzyl group, compound 18-a of the present invention can be produced by hydrogenation in the presence of a catalyst such as palladium carbon in an alcohol solvent such as methanol and ethanol, an ether solvent such as tetrahydrofuran and dioxane, a halogen solvent such as methylene chloride and chloroform, or an aromatic hydrocarbon solvent such as toluene and xylene.

Scheme 19

[Ka 22]

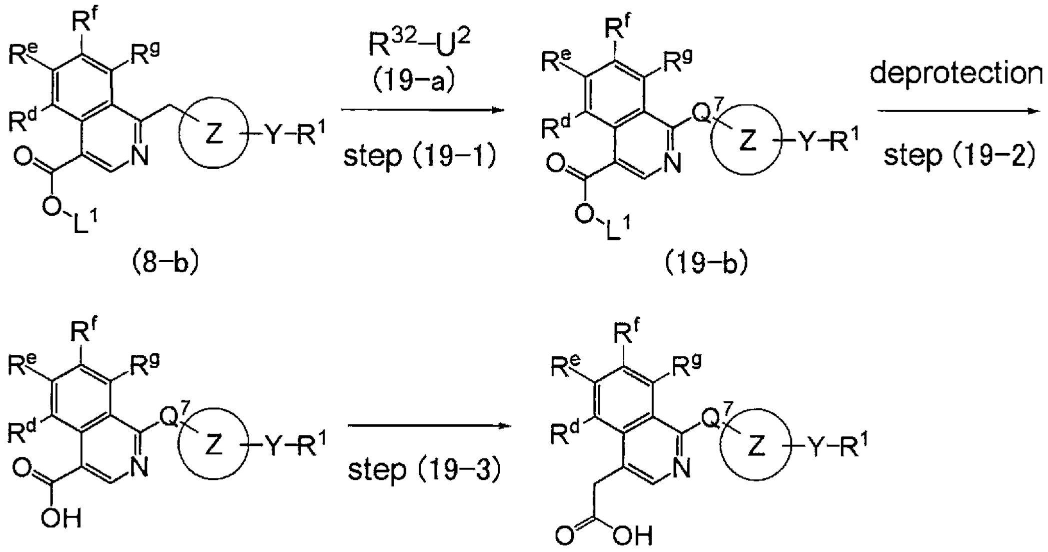


(8-b) (19-b)

(19-c) (19-d)

In the scheme, Z, Y, $R^1$, $R^d$, $R^e$, $R^f$ $R^g$, $L^1$, and $U^2$ are the same as defined above. $R^{32}$ represents a $C_{1-6}$ alkyl group, or a $C_{1-6}$ haloalkyl group; and Q7 represents the formula: - $CR^hR^i$- (wherein $R^h$ is a hydrogen atom, a $C_{1-6}$ alkyl group, or a $C_{1-6}$ haloalkyl group; and $R^i$ is a $C_{1-6}$alkyl group, or a $C_{1-6}$haloalkyl group.), or formula (II).

Step (19-1): Compound 19-b can be produced by using compound 8-b by the same procedure as used in step (12-1).

Step (19-2): Compound 19-c can be produced by using compound 19-b by the same procedure as used in step (1-2).

Step (19-3): Compound 19-d can be produced by using compound 19-c by the same procedure as used in step (1-3).

Scheme 20

[Ka 23]

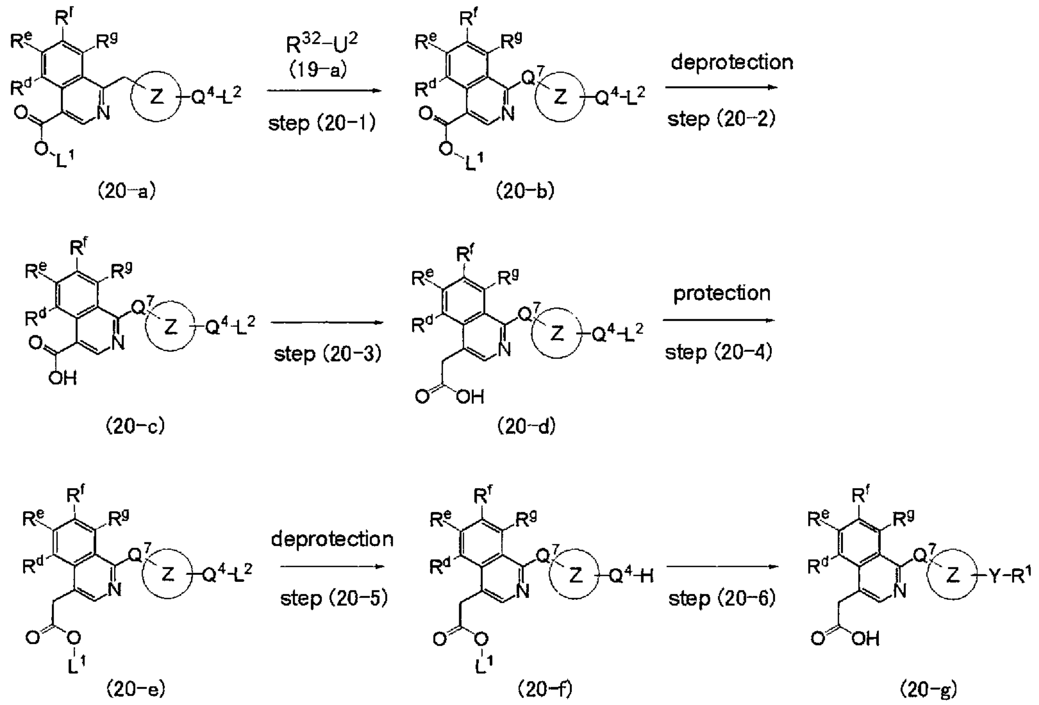


In the scheme, Z, Y, $R^1$, $R^d$, $R^e$, $R^f$, $R^g$, $L^1$, $L^2$, $Q^4$, $Q^7$, $R^{32}$, and $U^2$ are the same as defined above.
Step (20-1): Compound 20-b can be produced by using compound 20-a by the same procedure as used in step (19-1).
Step (20-2): Compound 20-c can be produced by using compound 20-b by the same procedure as used in step (1-2).
Step (20-3): Compound 20-d can be produced by using compound 20-c by the same procedure as used in step (1-3).
Step (20-4): Compound 20-e can be produced by using compound 20-d by the same procedure as used in step (2-4).
Step (20-5): Compound 20-f can be produced by using compound 20-e by the same procedure as used in step (2-5).

Step (20-6): Compound 20-g according to this invention can be produced by using compound 20-fby the same procedures as used in the steps in schemes 5 to 6. In this case, however, Y represents the formula: -O-W-, the formula: -O-W-O-, the formula: $-CH_2$-O-W-, the formula: -$CONR^3$-W-, the formula: -$CONR^3$-W-O-, or the formula: -$CONR^3$-W-$NR^4$CO-(W, $R^3$, and $R^4$ are the same as defined above).

Herein, the reaction is carried out in an appropriate temperature selected from - 78°C to boiling points of the solvents to be used in the reaction, and can be used at room temperature, under pressure, under irradiation with microwave, or the like.

Hereinafter, Examples and Test Examples are shown for describing the present invention in detail.

Examples

[Example 1]

{1-[1-(4-{[2-(4-chlorophenyl)ethyl]carbamoyl}phenyl)ethyl]isoquinolin-4-yl}acetic acid

[Ka 24]

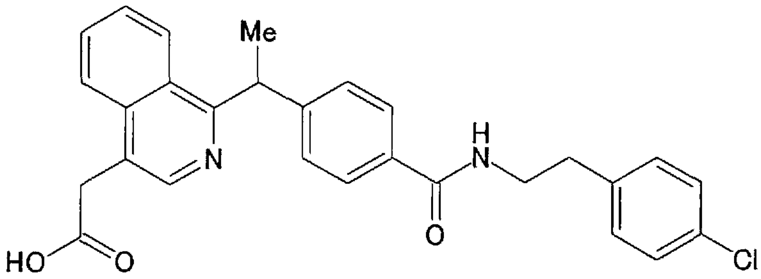

(1) To a solution of 4-(bromomethyl)benzoic acid tert-butyl ester (22.1 g) in toluene (440 ml), tri-n-butylphosphine (30.5 ml) was added, and the mixed solution was stirred at 60°C for 70 min. The reaction solution was removed by evaporation under reduced pressure, and to the resulting crude product, n-hexane was added and stirred. The resulting solid was filtered out to give [4-(tert-butoxycarbonyl)benzyl](tributyl)phosphonium bromide (36.0 g) as a colorless solid.

$^1$H NMR (600 MHz, CHLOROFORM-d) δ ppm 0.93 - 0.98 (m, 9 H), 1.44 - 1.52 (m, 12 H), 1.60 (s, 9 H), 2.37 - 2.46 (m, 6 H), 4.34 - 4.40 (m, 2 H), 7.50 - 8.02 (m, 4 H)

(2) To a solution of the compound (33.8 g) obtained in Example 1-(1) and 1-chloro-isoquinoline-4-carboxylic acid methyl ester (11.3 g) in tetrahydrofuran (235 ml), sodium bis(trimethylsilyl)amide (64 ml, 1.9 M solution) was added dropwise at -30°C, and the resulting solution was stirred at room temperature for 45 min. Furthermore, the resulting solution was stirred at 50°C for 75 min, and then a solution of sodium carbonate (10.2 g) in water (120 ml) was added, the resulting solution was stirred at 60°C for two hours. 1N aqueous hydrochloric acid solution was added so as to adjust the solution to pH 5, followed by extraction with ethyl acetate. The organic layer was dried over anhydrous magnesium sulfate, and evaporated under reduced pressure to remove the solvent. The resulting crude product was purified by column chromatography (neutral OH type silica gel, ethyl acetate / n-hexane = 10 to 30%) to give methyl 1-[4-(tert-butoxycarbonyl)benzyl]isoquinoline-4-carboxylate (12.0 g) as an orange-colored oily substance.

$^1$H NMR (600 MHz, CHLOROFORM-d) δ ppm 1.55 (s, 9 H), 4.02 (s, 3 H), 4.76 (s, 2 H), 7.27 - 8.98 (m, 8 H), 9.14 (s, 1 H)

(3) To a solution of the compound (0.217 g) obtained in Example 1-(2) in N,N-dimethylformamide (2 ml), sodium hydride (0.048 g) was added in an ice bath. The mixed solution was stirred for 5 min. Thereafter, methyl iodide (0.075 ml) was added thereto, and the mixed solution was stirred for 30 minutes in an ice bath. A saturated aqueous ammonium chloride solution was added, followed by extraction with ethyl acetate. Then, the organic layer was dried over anhydrous magnesium sulfate, and thereafter evaporated under reduced pressure to remove the solvent. The resulting crude product was purified by column chromatography (neutral OH type silica gel, ethyl acetate / n-hexane = 10%) to give methyl 1-{1-[4-(tert-butoxycarbonyl)phenyl]ethyl}isoquinoline-4-carboxylate (0.100 g) as a yellow oily substance. $^1$H NMR (600 MHz, CHLOROFORM-d) δ ppm 1.54 (s, 9 H), 1.84 (d, J = 6.9 Hz, 3 H), 4.02 (s, 3 H), 5.15 (q, J = 6.9 Hz, 1H), 7.31 - 9.22 (m, 9 H)

(4) To a solution of the compound (0.100 g) obtained in Example 1-(3) in tetrahydrofuran (1.3 ml), 1N aqueous sodium hydroxide solution (1.3 ml) was added in an ice bath, and the mixed solution was stirred for 11 hours at room temperature. 1 N aqueous hydrochloric acid solution was added thereto so as to adjust the solution to pH 2, and the pH-adjusted solution was extracted with ethyl acetate. The organic layer was washed with a saturated saline solution, dried over anhydrous magnesium sulfate, and thereafter evaporated under reduced pressure to remove the solvent to give 1-{1-[4-(tert-butoxycarbonyl)phenyl]ethyl}isoquinoline-4-carboxylic acid (0.096 g) as a yellow solid. $^1$H NMR (600 MHz, DMSO-$d_6$) δ ppm 1.48 (s, 9 H), 1.74 (d, J = 6.9 Hz, 3 H), 5.39 (q, J = 6.9 Hz, 1H), 7.41 -

9.08 (m, 9 H)

(5) To a solution of the compound (0.092 g) obtained in Example 1-(4) in chloroform (2 ml) were added oxalyl chloride (0.031 ml) and N,N-dimethylformamide (2 drops) in an ice bath, the mixed solution was stirred in an ice bath for 1 hour, and evaporated under reduced pressure to remove the solvent. To the resulting crude product, tetrahydrofuran (1 ml) and acetonitrile (1 ml) were added, and (trimethylsilyl)diazomethane (0.244 ml, 2 M solution) was added dropwise in an ice bath. The mixed solution was stirred in an ice bath for 1.5 hours. The solvent was removed by evaporation under reduced pressure, and to the resulting crude product, water (1 ml) and 1,4-dioxane (1 ml) and silver acetate (0.012 g) were added, and the mixed solution was stirred at 60°C for 50 minutes. The solution was returned to room temperature, and water was added to the solution, which was then extracted with ethyl acetate. The organic layer was washed with a saturated saline solution, dried over anhydrous magnesium sulfate, and evaporated under reduced pressure to remove the solvent. To the resulting crude product, methanol (2 ml) was added, and (trimethylsilyl)diazomethane (0.366 ml, 2 M solution) was added dropwise at room temperature, and the solution was stirred for 5 minutes. The solvent was removed by evaporation under reduced pressure, and the resulting crude product was purified by column chromatography (neutral OH type silica gel, ethyl acetate / n-hexane = 10 to 20%) to give tert-butyl 4-{1-[4-(2-methoxy-2-oxoethyl)isoquinolin-1-yl]ethyl}benzoate (0.048 g) as a yellow amorphous substance.

$^1$H NMR (600 MHz, CHLOROFORM-d) $\delta$ ppm 1.54 (s, 9 H), 1.83 (d, J = 6.9 Hz, 3 H), 3.69 (s, 3 H), 3.96 - 4.05 (m, 2 H), 5.10 (q, J = 6.9 Hz, 1 H), 7.32 - 8.49 (m, 9 H)

(6) To a solution of the compound (0.048 g) obtained in Example 1-(5) in chloroform (1 ml), trifluoroacetic acid (0.5 ml) was added in an ice bath, and the mixed solution was stirred for 5 hours at room temperature. A saturated aqueous sodium hydrogencarbonate solution was added thereto, and the resulting solution was extracted with ethyl acetate. The organic layer was washed with a saturated saline solution, dried over anhydrous magnesium sulfate, and thereafter evaporated under reduced pressure to remove the solvent to give 4-{ 1-[4-(2-methoxy-2-oxoethyl) isoquinolin-1-yl]ethyl}benzoic acid (0.062 g) as a pale yellow amorphous substance.

$^1$H NMR (600 MHz, CHLOROFORM-d) $\delta$ ppm 2.09 (d, J = 6.9 Hz, 3 H), 3.74 (s, 3 H), 4.18 (s, 2 H), 5.39 - 5.44 (m, 1 H), 7.40 - 8.71 (m, 9 H)

(7) To a solution of the compound (0.062 g) obtained in Example 1-(6) in chloroform (1 ml) were added 2-(4-chlorophenyl)ethylamine (0.022 g), triethylamine (0.050 ml), 1-hydroxybenzotriazole hydrate (0.027 g) and 1-ethyl-3-(dimethylaminopropyl)-carbodiimide hydrochloride (0.034 g), and the mixed solution was stirred for 16 hours at room temperature. A saturated aqueous ammonium chloride solution was added thereto, and the resulting solution was extracted with ethyl acetate. The organic layer was dried over anhydrous magnesium sulfate, and evaporated under reduced pressure to remove the solvent. The resulting crude product was purified by column chromatography (neutral OH type silica gel, ethyl acetate / n-hexane = 10 to 65%) to give {1-[1-(4-{[2-(4-chlorophenyl)ethyl]carbamoyl} phenyl)ethyl]isoquinolin-4-yl}acetate (0.030 g) as a colorless solid.

$^1$H NMR (600 MHz, CHLOROFORM-d) $\delta$ ppm 1.82 (d, J = 6.9 Hz, 3 H), 2.86 (t, J = 6.9 Hz, 2 H), 3.62 - 3.67 (m, 2 H), 3.70 (s, 3 H), 3.97 - 4.05 (m, 2 H), 5.04 - 5.16 (m, 1H), 5.95 - 6.02 (m, 1H), 7.10 - 8.51 (m, 13 H)

(8) To a solution of the compound (0.030 g) obtained in Example 1-(7) in tetrahydrofuran (0.5 ml) was added 1 N aqueous sodium hydroxide solution (0.3 ml) in an ice bath, and the mixed solution was stirred for 2 hours at room temperature. 1 N aqueous hydrochloric acid solution was added to the solution in an ice bath to adjust the solution to pH 3, which was then extracted with ethyl acetate. The organic layer was washed with a saturated saline solution, dried over anhydrous magnesium sulfate, and thereafter evaporated under reduced pressure to remove the solvent. The resulting crude product was purified by column chromatography (acidic OH type silica gel, ethyl acetate / n-hexane = 30 to 75%). To the obtained solid, diethyl ether was added, and the mixture was stirred at room temperature for 17 hours and was filtered out to give the title compound (0.030 g) as a colorless solid. $^1$H NMR (600 MHz, DMSO-$d_6$) $\delta$ ppm 1.71 (d, J = 6.9 Hz, 3 H), 2.77 (t, J = 7.1 Hz, 2 H), 3.35 - 3.45 (m, 2 H), 3.98 (s, 2 H), 5.26 (q, J = 6.9 Hz, 1H), 7.20 - 8.43 (m, 14 H)

[Example 2]

{1-[(4-{[2-(4-chlorophenyl)ethyl]carbamoyl}phenyl)(difluoro)methy]isoquinolin-4-yl}acetic acid

[Ka 25]

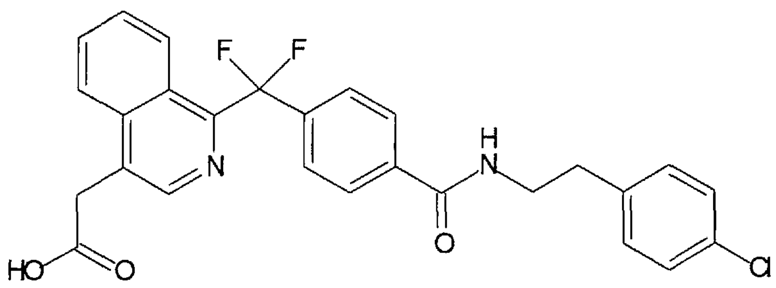

(1) To a solution of difluoro[4-(methoxycarbonyl)phenyl]acetic acid (1.54 g) in thionyl chloride (15 ml) was added N, N-dimethylformamide (2 drops) in an ice bath, and the mixed solution was stirred for 1hour at room temperature, then for 1hour at 50°C, and thereafter for 2 hours at 85°C. The resulting solution was evaporated under reduced pressure to remove the solvent. To a solution of the resulting crude product in chloroform (35 ml) were added 4-dimethyl amino pyridine (0.082 g), triethylamine (6.7 ml), and ethyl 4-amino-3-phenylbutanoate hydrochloride (1.80 g), and the mixed solution was stirred at room temperature overnight. Saturated aqueous ammonium chloride solution was added to the solution, and the solution was extracted with ethyl acetate. The organic layer was dried over anhydrous sodium sulfate, and was evaporated under reduced pressure to remove the solvent. The resulting crude product was purified by column chromatography (neutral OH type silica gel, ethyl acetate / n-hexane = 25 to 33%) to give methyl 4-{2-[(4-ethoxy-4-oxo-2-phenylbutyl)amino]-1,1-difluoro-2-oxoethyl}benzoate (2.00 g).

$^1$H NMR (600 MHz, CHLOROFORM-d) $\delta$ ppm 1.13 - 1.19 (m, 3 H), 2.64 - 2.68 (m, 2 H), 3.34 -3.41 (m, 1H), 3.48 - 3.55 (m, 1H), 3.65 - 3.71 (m, 1 H), 3.93 - 3.95 (m, 3 H), 4.04 - 4.09 (m, 2 H), 6.56 (br. s., 1H), 7.13 - 8.08 (m, 9 H)

(2) To a solution of the compound (1.77 g) obtained in Example 2-(1) in chloroform (42 ml) was added 2-chloropyridine (0.594 ml), and then was added trifluoromethanesulfonic anhydride (1.0 ml) by drops at -60°C. The reaction solution was stirred for 5 minutes at -60°C, then for 5 minutes in an ice bath, thereafter for 5 minutes at room temperature, and further for 30 minutes at 100°C. Saturated aqueous sodium hydrogen carbonate solution was added to the resulting solution, and was extracted with chloroform. The organic layer was dried over anhydrous sodium sulfate, and then evaporated under reduced pressure to remove the solvent. To the resulting crude product, sulfur (0.155 g) was added, and the mixed solution was stirred at 165°C for 2.5 hours. Ethanol was added to the resulting solution, which was then subjected to filtration. The filtrate was concentrated by evaporation under reduced pressure. The obtained crude product was purified by column chromatography (neutral OH type silica gel, ethyl acetate / n-hexane = 10 to 20%) to give methyl 4-{[4-(2-ethoxy-2-oxoethyl)isoquinolin-1-yl](difluoro)methyl}benzoate (0.505 g).

$^1$H NMR (600 MHz, CHLOROFORM-d) $\delta$ ppm 1.23 (t, J = 7.0 Hz, 3 H), 3.94 (s, 3 H), 4.04 (s, 2 H), 4.16 (q, J = 7.0 Hz, 2 H), 7.61 - 8.48 (m, 9 H)

(3) To a solution of the compound (0.500 g) obtained in Example 2-(2) in tetrahydrofuran (17.5 ml) was added 2 N aqueous sodium hydroxide solution (2. 5 ml), and the mixed solution was stirred at room temperature overnight. The resulting solution was evaporated under reduced pressure to remove the solvent. To the resulting crude product, 2 N aqueous hydrochloric acid solution was added, and the resulting solution was extracted with chloroform. The organic layer was dried over anhydrous sodium sulfate, and evaporated under reduced pressure to remove the solvent to give 4-{[4-(carboxymethyl)isoquinolin-1-yl](difluoro)methyl}benzoic acid (0.382 g).

$^1H$ NMR (600 MHz, DMSO-$d_6$) δ ppm 4.13 (s, 2 H), 7.70 - 8.46 (m, 9 H)

(4) To a solution of the compound (0.367 g) obtained in Example 2-(3) in methanol (10 ml) was added thionyl chloride (0.004 ml), and the mixed solution was stirred for 2 days at room temperature. The resulting solution was evaporated under reduced pressure to remove the solvent. The obtained crude product was purified by column chromatography (neutral OH type silica gel, methanol / chloroform = 3 to 9%) to give 4- {difluoro[4-(2-methoxy-2-oxoethyl)isoquinolin-1-yl]methyl}benzoic acid (0.380 g).

$^1H$ NMR (600 MHz, DMSO-$d_6$) δ ppm 3.63 (s, 3 H), 4.25 (s, 2 H), 7.68 - 8.48 (m, 9 H)

(5) To a solution of the compound (0.079 g) obtained in Example 2-(4) in chloroform (1.1 ml) were added 2-(4-chlorophenyl)ethylamine (0.038 ml), 1-hydroxybenzotriazole hydrate (0.037 g) and 1-ethyl-3-(dimethylaminopropyl)-carbodiimide hydrochloride (0.053 g). The mixed solution was stirred at room temperature overnight. Water was added to the resulting solution, which was then extracted with chloroform. The organic layer was washed with saturated saline solution, then dried over anhydrous sodium sulfate, and evaporated under reduced pressure to remove the solvent. The resulting crude product was purified by column chromatography (neutral OH type silica gel, ethyl acetate / n-hexane = 33 to 50%) to give methyl {1-[(4-{[2-(4-chlorophenyl)ethyl]carbamoyl}phenyl)(difluoro)methyl]isoquinolin-4-yl}acetate (0.060 g).
$^1H$ NMR (600 MHz, CHLOROFORM-d) δ ppm 2.91 (t, J=6.9 Hz, 2 H), 3.68 - 3.73 (m, 5 H), 4.05 (s, 2 H), 6.09 - 6.16 (m, 1H), 7.15 - 8.48 (m, 13 H)

(6) To a solution of the compound (0.060 g) obtained in Example 2-(5) in tetrahydrofuran (0.9 ml) was added 2 N aqueous sodium hydroxide solution (0.3 ml) in an ice bath, and the mixed solution was stirred for 4.5 hours at room temperature. The resulting solution was evaporated under reduced pressure to remove the solvent. To the obtained crude product was added saturated aqueous ammonium chloride solution, and the resulting solution was extracted with chloroform. The organic layer was evaporated under reduced pressure to remove the solvent. The resulting crude product was purified by column chromatography (neutral OH type silica gel, methanol / chloroform = 3 to 9%) to give the title compound (0.046 g).

$^1H$ NMR (600 MHz, DMSO-$d_6$) δ ppm 2.84 (t, J = 7.3 Hz, 2 H), 3.46 - 3.51 (m, 2 H), 4.13 (s, 2 H), 7.24 - 8.69 (m, 14 H)

[Example 3]

<u>[1-(4-{[2-(4-chlorophenyl)ethyl]carbamoyl}-3-methoxybenzyl)isoquinolin-4-yl]acetic acid</u>

[Ka 26]

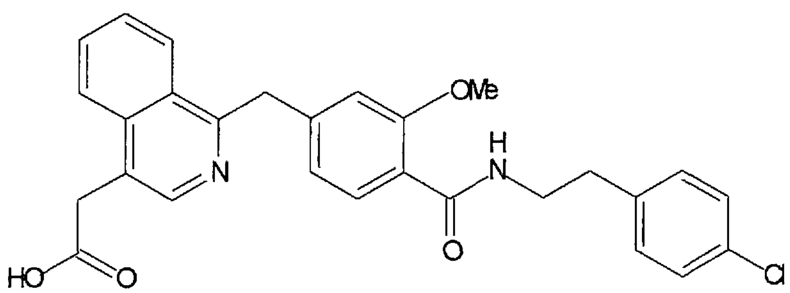

(1) To a solution of 2-methoxy-4-methylbenzoyl chloride (2.50 g) in chloroform (3 ml) were added oxalyl chloride

(1.9 ml) and N,N-dimethylformamide (2 drops) in an ice bath, the mixed solution was stirred for 3.5 hours at room temperature. The solvent was removed by evaporation under reduced pressure. To a solution of the obtained crude product in tetrahydrofuran (5 ml) were added pyridine (1.82 ml) and tert-butyl alcohol (2.2 ml), and the resulting solution was stirred for 19 hours at 85°C. The solution was returned to room temperature, and water was added to the solution, which was then extracted with ethyl acetate. The organic layer was washed successively with 10% sulfuric acid, 10% aqueous sodium hydroxide solution, and water, dried over anhydrous magnesium sulfate, and evaporated under reduced pressure to remove the solvent to give tert-butyl 2-methoxy-4-methylbenzoate (3.21 g) as an orange oily substance.

$^1$H NMR (600 MHz, CHLOROFORM-d) δ ppm 1.57 (s, 9 H), 2.37 (s, 3 H), 3.88 (s, 3 H), 6.74 - 6.77 (m, 2 H), 7.65 - 7.67 (m, 1H)

(2) To a solution of the compound (1.00 g) obtained in Example 3-(1) in chloroform (10 ml) were added N-bromosuccinimide (0.881 g) and azobisisobutyronitrile (0.050 g), and the mixed solution was heated at reflux for 1 hour. The resulting solution was filtered. The filtrate was washed with water, dried over anhydrous magnesium sulfate and evaporated under reduced pressure to remove the solvent to give tert-butyl 4-(bromomethyl)-2-methoxybenzoate (1.57 g) as a pale yellow oily substance.

$^1$H NMR (600 MHz, CHLOROFORM-d) δ ppm 1.58 (s, 9 H), 3.91 (s, 3 H), 4.4.6 (s, 2 H), 6.94 - 7.71 (m, 3 H)

(3) The same procedure as used in Example 1-(1) was carried out using the compound (0.814 g) obtained in Example 3-(2) to give [4-(tert-butoxycarbonyl)-3-methoxybenzyl](tributyl)phosphonium bromide (0.897 g) as a colorless solid. $^1$H NMR (600 MHz, CHLOROFORM-d) δ ppm 0.93 - 0.98 (m, 9 H), 1.39 - 1.53 (m, 12 H), 1.58 (s, 9 H), 2.34 - 2.47 (m, 6 H), 3.97 (s, 3 H), 4.30 - 4.36 (m, 2 H), 6,77 - 7.73 (m, 3 H)

(4) The same procedure as used in Example 1-(2) was carried out using the compound (0.500 g) obtained in Example 3-(3) to give methyl 1-[4-(tert-butoxycarbonyl)-3-methoxybenzyl]isoquinoline-4-carboxylate (0.096 g) as an orange oily substance. $^1$H NMR (600 MHz, CHLOROFORM-d) δ ppm 1.54 (s, 9 H), 3.79 (s, 3 H), 4.03 (s, 3 H), 4.71 (s, 2 H), 6.80 - 9.14 (m, 8 H)

(5) The same procedure as used in Example 1-(4) was carried out using the compound (0.096 g) obtained in Example 3-(4) to give 1-[4-(tert-butoxycarbonyl)-3-methoxybenzyl]isoquinoline-4-carboxylic acid (0.098 g) as a yellow amorphous substance. $^1$H NMR (600 MHz, DMSO-$d_6$) δ ppm 1.46 (s, 9 H), 3.76 (s, 3 H), 4.75 (s, 2 H), 6.78 - 9.02 (m, 8 H)

(6) The same procedure as used in Example 1-(S) was carried out using the compound (0.098 g) obtained in Example 3-(5) to give tert-butyl 2-methoxy-4-{[4-(2-methoxy-2-oxoethyl)isoquinolin-1-yl]methyl}benzoate (0.024 g) as an orange oily substance. $^1$H NMR (600 MHz, CHLOROFORM-d) δ ppm 1.53 (s, 9 H), 3.69 (s, 3 H), 3.80 (s, 3 H), 4.01 (s, 2 H), 4.66 (s, 2 H), 6.80 - 8.42 (m, 8 H)

(7) The same procedure as used in Example 1-(6) was carried out using the compound (0.024 g) obtained in Example 3-(6) to give 2-methoxy-4-{[4-(2-methoxy-2-oxoethyl)isoquinolin-1-yl]methyl}benzoic acid (0.021 g) as an orange oily substance. $^1$H NMR (600 MHz, DMSO-$d_6$) δ ppm 3.62 (s, 3H), 3.78 (s, 3 H), 4.15 (s, 2 H), 4.69 (br. s., 2 H), 6.78 - 8.41 (m, 8 H)

(8) To a solution of the compound (0.021 g) obtained in Example 3-(7) in tetrahydrofuran (1.5 ml) were added 2-(4-chlorophenyl)ethylamine (0.016 ml), 1-hydroxybenzotriazole hydrate (0.017 g) and 1-ethyl-3-(dimethylaminopropyl)-carbodiimide hydrochloride (0.022 g), and the mixed solution was stirred for 9.5 hours at room temperature. Water was added thereto, and the resulting solution was extracted with ethyl acetate. The organic layer was dried over anhydrous magnesium sulfate, and evaporated under reduced pressure to remove the solvent. The resulting crude product was purified by column chromatography (NH type silica gel, ethyl acetate / n-hexane = 10 to 50%) to give methyl [1-(4-{[2-(4-chlorophenyl)ethyl]carbamoyl}-3-methoxybenzyl)isoquinolin-4-yl]acetate (0.010 g) as an orange oily substance.

$^1$H NMR (600 MHz, CHLOROFORM-d) δ ppm 2.86 (t, J = 6.7 Hz, 2 H), 3.66 - 3.69 (m, 5 H), 3.70 (s, 3 H), 4.01 (s, 2 H), 4.66 (s, 2 H), 6.84 - 8.41 (m, 13 H)

(9) The same procedure as used in Example 1-(8) was carried out using the compound (0.010 g) obtained in Example 3-(8) to give the title compound (0.005 g) as a pale yellow solid.

$^1$H NMR (600 MHz, DMSO-$d_6$) δ ppm 2.79 (t, J = 7.1 Hz, 2 H), 3.43 - 3.48 (m, 2 H), 3.77 (s, 3 H), 4.01 (s, 2 H), 4.65 (s, 2 H), 6.83 - 8.39 (m, 13 H)

[Example 4]

<u>[1-(4-{[2-(4-chlorophenyl)ethyl]carbamoyl}-2-fluorobenzyl)isoquinolin-4-yl]acetic acid</u>

[Ka 27]

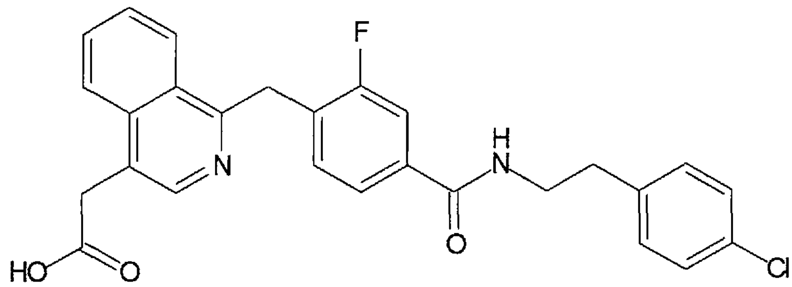

(1) The same procedure as used in Example 3-(2) was carried out using tert-butyl 3-fluoro-4-methylbenzoate (1.41 g) to give tert-butyl 4-(bromomethyl)-3-fluorobenzoate (2.10 g) as a yellow oily substance.

$^1$H NMR (600 MHz, CHLOROFORM-d) δ ppm 1.59 (s, 9 H), 4.51 (s, 2 H), 7.42 - 7.46 (m, 1 H), 7.64 - 7.68 (m, 1H), 7.74 - 7.77 (m, 1H)

(2) The same procedure as used in Example 1-(1) was carried out using the compound (2.10 g) obtained in Example 4-(1) to give [4-(tert-butoxycarbonyl)-2-fluorobenzyl](tributyl)phosphonium bromide (2.94 g) as a colorless oily substance.

$^1$H NMR (600 MHz, CHLOROFORM-d) δ ppm 0.95 (t, J = 7.1 Hz, 9 H), 1.43 - 1.54 (m, 12 H), 1.60 (s, 9 H), 2.42 - 2.49 (m, 6 H), 4.44 (d, J = 16.1 Hz, 2 H), 7.69 - 8.15 (m, 3 H)

(3) The same procedure as used in Example 1-(2) was carried out using the compound (2.94 g) obtained in Example 4-(2) to give methyl 1-[4-(tert-butoxycarbonyl)-2-fluorobenzyl]isoquinoline-4-carboxylate (0.228 g) as an orange oily substance.

$^1$H NMR (600 MHz, CHLOROFORM-d) δ ppm 1.56 (s, 9 H), 4.02 (s, 3 H), 4.76 (s, 2 H), 7.10 - 9.12 (m, 8H)

(4) The same procedure as used in Example 1-(4) was carried out using the compound (0.228 g) obtained in Example 4-(3) to give 1-[4-(tert-butoxycarbonyl)-2-fluorobenzyl]isoquinoline-4-carboxylic acid (0.205 g) as a yellow solid.

$^1$H NMR (600 MHz, DMSO-$d_6$) δ ppm 1.54 (s, 9 H), 4.83 (s, 2 H), 7.33 - 8.95 (m, 8 H)

(5) The same procedure as used in Example 1-(5) was carried out using the compound (0.205 g) obtained in Example 4-(4) to give tert-butyl 3-fluoro-4-{[4-(2-methoxy-2-oxoethyl)isoquinolin-1-yl]methyl}benzoate (0.036 g) as a yellow oily substance.

$^1$H NMR (600 MHz, CHLOROFORM-d) δ ppm 1.54 (s, 9 H), 3.69 (s, 3 H), 4.01 (s, 2 H), 4.70 (br. s., 2 H), 7.54 - 8.42 (m, 8 H)

(6) The same procedure as used in Example 1-(6) was carried out using the compound (0.036 g) obtained in Example 4-(5) to give 3-fluoro-4-{[4-(2-methoxy-2-oxoethyl)isoquinolin-1-yl]methyl}benzoic acid (0.022 g) as a yellow oily substance.

$^1$H NMR (600 MHz, DMSO-$d_6$) δ ppm 3.61 (s, 3 H), 4.13 (s, 2 H), 4.75 (s, 2 H), 7.32 - 8.37 (m, 8 H)

(7) The same procedure as used in Example 3-(8) was carried out using the compound (0.022 g) obtained in Example 4-(6) to give methyl [1-(4-{[2-(4-chlorophenyl)ethyl]carbamoyl}-2-fluorobenzyl)isoquinolin-4-yl]acetate (0.012 g) as a yellow solid.

$^1$H NMR (600 MHz, CHLOROFORM-d) δ ppm 2.88 (t, J = 6.9 Hz, 2 H), 3.64 - 3.68 (m, 2 H), 3.70 (s, 3 H), 4.01 (s, 2 H), 4.69 (s, 2 H), 5.95 - 6.02 (m, 1 H), 7.12 - 8.40 (m, 12 H)

(8) The same procedure as used in Example 1-(8) was carried out using the compound (0.012 g) obtained in Example 4-(7) to give the title compound (0.005 g) as a pale yellow solid.

$^1$H NMR (600 MHz, DMSO-$d_6$) δ ppm 2.82 (t, J = 7.1 Hz, 2 H), 3.44 - 3.50 (m, 2 H), 3.98 (s, 2 H), 4.70 (s, 2 H), 7.23 - 8.58 (m, 13 H)

[Example 5]

<u>[1-(4-{[2-(4-chlorophenyl)ethyl]carbamoyl}-3-methylbenzyl)isoquinolin-4-yl]acetic acid</u>

[Ka 28]

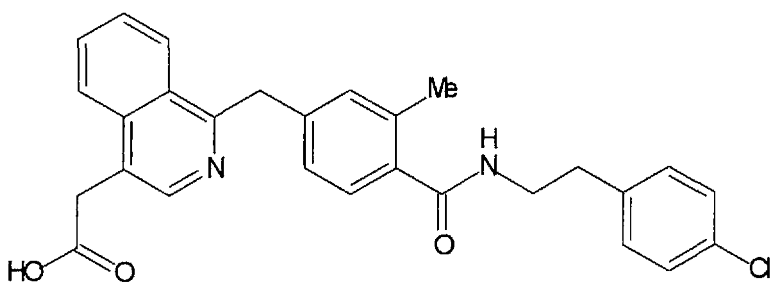

(1) To a solution of tert-butyl 4-formyl-2-methylbenzoate (2.76 g) in methanol (140 ml) was added sodium borohydride (0.474 g) in an ice bath, and the mixed solution was stirred for 15 minutes. A saturated aqueous ammonium chloride solution was added thereto, and the resulting solution was extracted with ethyl acetate. The organic layer was dried over anhydrous magnesium sulfate, and evaporated under reduced pressure to remove the solvent. The resulting crude product was purified by column chromatography (neutral OH type silica gel, ethyl acetate / n-hexane = 10 to 2%) to give tert-butyl 4-(hydroxymethyl)-2-methylbenzoate (1.22 g) as a colorless oily substance.

$^1$H NMR (600 MHz, CHLOROFORM-d) δ ppm 1.59 (s, 9 H), 2.58 (s, 3 H), 4.70 (s, 2 H), 7.19 - 7.83 (m, 3 H)

(2) To a solution oftriphenyl phosphine (1.72 g) and carbon tetrabromide (2.18 g) in tetrahydrofuran (30 ml) was added a solution of the compound (1.22 g) obtained in Example 5-(1) in tetrahydrofuran (6 ml). The mixed solution was stirred for 2 hours at room temperature. Triphenyl phosphine (0.431 g) and carbon tetrabromide (0.544 g) were further added thereto. The resulting solution was stirred for 30 minutes at room temperature, and evaporated under reduced pressure to remove the solvent. The resulting crude product was purified by column chromatography (neutral OH type silica gel, n-hexane) to give tert-butyl 4-(bromomethyl)-2-methylbenzoate (1.30 g) as a colorless oily substance.

$^1$H NMR (600 MHz, CHLOROFORM-d) δ ppm 1.59 (s, 9 H), 2.56 (s, 3 H), 4.44 (s, 2 H), 7.21 - 7.81 (m, 3 H)

(3) The same procedure as used in Example 1-(1) was carried out using the compound (1.30 g) obtained in Example 5-(2) to give [4-(tert-butoxycarbonyl)-3-methylbenzyl](tributyl)phosphonium bromide (2.12 g) as a colorless solid.

$^1$H NMR (600 MHz, CHLOROFORM-d) δ ppm 0.95 (t, J = 6.9 Hz, 9 H), 1.44 - 1.53 (m, 12 H), 1.60 (s, 9 H), 2.39 - 2.46 (m, 6 H), 2.56 (s, 3 H), 4.27 (d, J = 15.6 Hz, 2 H), 7.26 - 7.83 (m, 3 H)

(4) The same procedure as used in Example 1-(2) was carried out using the compound (2.12 g) obtained in Example 5-(3) to give methyl 1-[4-(tert-butoxycarbonyl)-3-methylbenzyl]isoquinoline-4-carboxylate (0.388 g) as an orange oily substance.

$^1$H NMR (600 MHz, CHLOROFORM-d) δ ppm 1.55 (s, 9 H), 2.49 (s, 3 H), 4.03 (s, 3 H), 4.69 (s, 2 H), 7.06 - 9.16 (m, 8 H)

(5) The same procedure as used in Example 1-(4) was carried out using the compound (0.388 g) obtained in Example 5-(4) to give 1-[4-(tert-butoxycarbonyl)-3-methylbenzyl]isoquinoline-4-carboxytic acid (0.345 g) as a pale yellow solid.

$^1$H NMR (600 MHz, DMSO-$d_6$) δ ppm 1.50 (s, 9 H), 2.41 (s, 3 H), 4.72 (s, 2 H), 7.15 - 9.02 (m, 8 H)

(6) The same procedure as used in Example 1-(5) was carried out using the compound (0.345 g) obtained in Example 5-(5) to give tert-butyl 4-{[4-(2-methoxy-2-oxoethyl)isoquinolin-1-yl]methyl}-2-methylbenzoate (0.127 g) as an orange oily substance.

$^1$H NMR (600 MHz, CHLOROFORM-d) δ ppm 1.55 (s, 9 H), 2.50 (s, 3 H), 3.70 (s, 3 H), 4.01 (s, 2 H), 4.64 (s, 2 H), 7.08 - 8.43 (m, 8 H)

(7) The same procedure as used in Example 1-(6) was carried out using the compound (0.127 g) obtained in Example 5-(6) to give 4-{[4-(2-methoxy-2-oxoethyl)isoquinolin-1-yl]methyl}-2-methylbenzoic acid (0.111 g) as a yellow solid.

$^1$H NMR (600 MHz, DMSO-$d_6$) δ ppm 2.45 (s, 3 H), 3.62 (s, 3 H), 4.18 (s, 2 H), 4.70 (s, 2 H), 7.17 - 8.44 (m, 8 H)

(8) The same procedure as used in Example 3-(8) was carried out using the compound (0.111 g) obtained in Example 5-(7) to give methyl [1-(4-{[2-(4-chlorophenyl)ethyl]carbamoyl}-3-methylbenzyl)isoquinolin-4-yl]acetate (0.072 g) as a pale yellow solid.

$^1$H NMR (600 MHz, CHLOROFORM-d) δ ppm 2.32 (s, 3 H), 2.87 (t, J = 6.7 Hz, 2 H), 3.63 - 3.68 (m, 2 H), 3.70 (s, 3 H), 4.01 (s, 2 H), 4.61 (s, 2 H), 7.03 - 8.42 (m, 12 H)

(9) The same procedure as used in Example 1-(8) was carried out using the compound (0.072 g) obtained in Example 5-(8) to give the title compound (0.050 g) as a pale yellow solid.

$^1$H NMR (600 MHz, DMSO-$d_6$) δ ppm 2.16 (s, 3 H), 2.76 (t, J = 7.1 Hz, 2 H), 3.38 - 3.42 (m, 2 H), 4.01 (s, 2 H), 4.60 (s, 2 H), 7.08 - 8.38 (m, 13 H), 12.52 (br. s., 1 H)

[Example 6]

<u>[1-(4-{[2-(4-chlorophenyl)ethyl]carbamoyl}-3-fluorobenzyl)isoquinolin-4-yl]acetic acid</u>

[Ka 29]

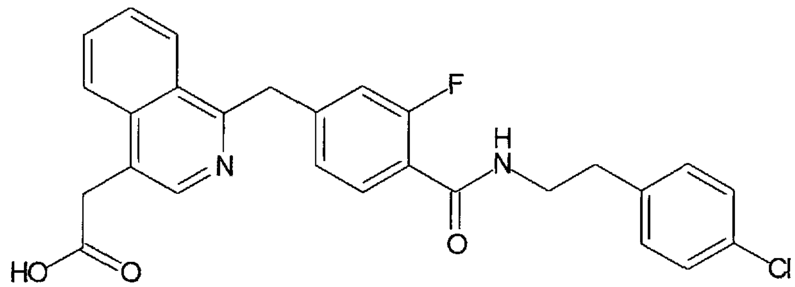

(1) To a solution of 2-fluoro-4-methylbenzoic acid (2.62 g) in toluene (34 ml) were added N,N-dimethylformamide di-tert-butylacetal (16.3 ml), the mixed solution was stirred for 1 hour at 100°C. The solution was returned to room temperature, and water was added to the solution, which was then extracted with ethyl acetate. The organic layer was washed successively with a saturated aqueous sodium hydrogen carbonate solution and a saturated saline solution, dried over anhydrous magnesium sulfate, and evaporated under reduced pressure to remove the solvent. The resulting crude product was purified by column chromatography (neutral OH type silica gel, ethyl acetate / n-hexane = 0 to 20%) to give tert-butyl 2-fluoro-4-methylbenzoate (2.87 g) as a pale yellow oily substance.

$^1$H NMR (600 MHz, CHLOROFORM-d) δ ppm 1.59 (s, 9 H), 2.37 (s, 3 H), 6.89 - 7.77 (m, 3 H)

(2) The same procedure as used in Example 3-(2) was carried out using the compound (2.87 g) obtained in Example 6-(1) to give tert-butyl 4-(bromomethyl)-2-fluorobenzoate (4.31 g) as a pale yellow oily substance.

$^1$H NMR (600 MHz, CHLOROFORM-d) δ ppm 1.59 (s, 9 H), 4.43 (s, 2 H), 7.12 - 7.85 (m, 3 H)

(3) The same procedure as used in Example 1-(1) was carried out using the compound (4.31 g) obtained in Example 6-(2) to give [4-(tert-butoxycarbonyl)-3-fluorobenzyl](tributyl)phosphonium bromide (5.41 g) as a pale pink solid.

$^1$H NMR (600 MHz, CHLOROFORM-d) δ ppm 0.93 - 0.97 (m, 9 H), 1.45 - 1.52 (m, 12 H), 1.59 (s, 9 H), 2.38 - 2.48 (m, 6 H), 4.52 (d, J = 16.1 Hz, 2 H), 7.23 - 7.90 (m, 3 H)

(4) The same procedure as used in Example 1-(2) was carried out using the compound (4.10 g) obtained in Example 6-(3) to give methyl 1-[4-(tert-butoxycarbonyl)-3-fluorobenzyl]isoquinoline-4-carboxylate (1.17 g) as an orange solid.

$^1$H NMR (600 MHz, CHLOROFORM-d) δ ppm 1.55 (s, 9 H), 4.72 (s, 2 H), 6.95 - 9.17 (m, 8 H)

(5) The same procedure as used in Example 1-(4) was carried out using the compound (1.17 g) obtained in Example 6-(4) to give 1-[4-(tert-butoxycarbonyl)-3-fluorobenzyl]isoquinotine-4-carboxylic acid (1.08 g) as an orange solid.

$^1$H NMR (600 MHz, DMSO-$d_6$) δ ppm 1.50 (s, 9 H), 4.80 (s, 2 H), 7.17 - 9.00 (m, 8 H)

(6) The same procedure as used in Example 1-(5) was carried out using the compound (0.600 g) obtained in Example 6-(5) to give tert-butyl 2-fluoro-4-{[4-(2-methoxy-2-oxoethyl)isoquinolin-1-yl]methyl}benzoate (0.152 g) as a brown amorphous substance.

$^1$H NMR (600 MHz, CHLOROFORM-d) δ ppm 1.55 (s, 9 H), 3.70 (s, 3 H), 4.02 (s, 2 H), 4.67 (s, 2 H), 6.96 - 8.43 (m, 8 H)

(7) The same procedure as used in Example 1-(6) was carried out using the compound (0.152 g) obtained in Example 6-(6) to give 2-fluoro-4-{[4-(2-methoxy-2-oxoethyl)isoquinolin-1-yl]methyl}benzoic acid (0.177 g) as a yellow solid.

$^1$H NMR (600 MHz, DMSO-$d_6$) δ ppm 3.64 (s, 3 H), 4.24 (br. s., 2 H), 4.83 (br. s., 2 H), 7.19 - 8.51 (m, 8H)

(8) The same procedure as used in Example 3-(8) was carried out using the compound (0.155 g) obtained in Example 6-(7) to give methyl [1-(4-{[2-(4-chlorophenyl)ethyl]carbamoyl}-3-fluorobenzyl)isoquinolin-4-yl]acetate (0.074 g) as a yellow solid.

$^1$H NMR (600 MHz, CHLOROFORM-d) δ ppm 2.87 (t, J = 6.9 Hz, 2 H), 3.65 - 3.69 (m, 2 H), 3.70 (s, 3 H), 4.02 (s, 2 H), 4.67 (s, 2 H), 6.62 - 6.69 (m, 1H), 6.93 - 8.43 (m, 12 H)

(9) The same procedure as used in Example 1-(8) was carried out using the compound (0.074 g) obtained in Example 6-(8) to give the title compound (0.052 g) as a pale yellow solid.

$^1$H NMR (600 MHz, DMSO-$d_6$) δ ppm 2.78 (t, J = 7.1 Hz, 2 H), 3.39 - 3.44 (m, 2 H), 4.02 (s, 2 H), 4.68 (s, 2 H), 7.16 - 8.39 (m, 13 H), 12.53 (br. s., 1H)

[Example 7]

{1-[4-{[2-(4-chlorophenyl)ethyl]carbamoyl}-3-(trifluoromethyl)benzyl]isoquinolin-4-yl}acetic acid

[Ka 30]

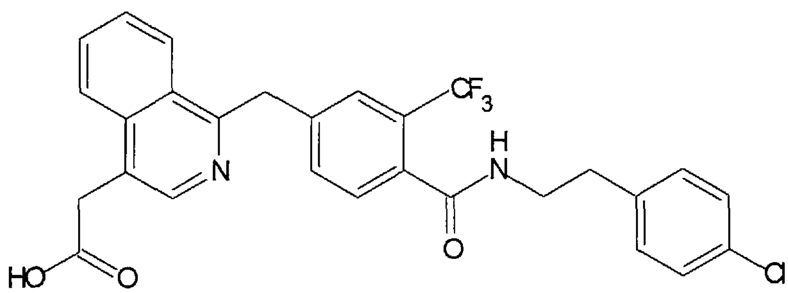

(1) The same procedure as used in Example 6-(1) was carried out using 4-methyl-2-(trifluoromethyl)benzoic acid (2.04 g) to give tert-butyl 4-methyl-2-(trifluoromethyl)benzoate (2.38 g) as a colorless oily substance.

$^1$H NMR (600 MHz, CHLOROFORM-d) δ ppm 1.58 (s, 9 H), 2.43 (s, 3 H), 7.35 - 7.65 (m, 3 H)

(2) The same procedure as used in Example 3-(2) was carried out using the compound (2.38 g) obtained in Example 7-(1) to give tert-butyl 4-(bromomethyl)-3-(trifluoromethyl)benzoate (3.02 g) as a colorless amorphous substance.

$^1$H NMR (600 MHz, CHLOROFORM-d) δ ppm 1.58 (s, 9 H), 4.49 (s, 2 H), 7.58 - 7.73 (m, 3 H)

(3) The same procedure as used in Example 1-(1) was carried out using the compound (3.02 g) obtained in Example 7-(2) to give [4-(tert-butoxycarbonyl)-3-(trifluoromethyl)benzyl](tributyl)phosphonium bromide (3.60 g) as a colorless solid.

$^1$H NMR (600 MHz, CHLOROFORM-d) δ ppm 0.91 - 0.96 (m, 9 H), 1.43 - 1.53 (m, 12 H), 1.59 (s, 9 H), 2.37 - 2.47 (m, 6 H), 4.63 (d, J = 16.1 Hz, 2 H), 7.62 - 8.07 (m, 3 H)

(4) The same procedure as used in Example 1-(2) was carried out using the compound (1.03 g) obtained in Example 7-(3) to give methyl 1-[4-(tert-butoxycarbonyl)-3-(trifluoromethyl)benzyl]isoquinoline-4-carboxylate (0.188 g) as an orange oily substance.

$^1$H NMR (600 MHz, CHLOROFORM-d) δ ppm 1.55 (s, 9 H), 4.03 (s, 3 H), 4.77 (s, 2 H), 7.41 - 9.15 (m, 8 H)

(5) The same procedure as used in Example 1-(4) was carried out using the compound (0.188 g) obtained in Example 7-(4) to give 1-[4-(tert-butoxycarbonyl)-3-(trifluoromethyl)benzyl]isoquinoline-4-carboxylic acid (0.182 g) as a pale yellow solid.

$^1$H NMR (600 MHz, DMSO-$d_6$) δ ppm 1.50 (s, 9 H), 4.89 (s, 2 H), 7.62 - 8.99 (m, 8 H)

(6) The same procedure as used in Example 1-(5) was carried out using the compound (0.182 g) obtained in Example 7-(5) to give tert-butyl 4-{[4-(2-methoxy-2-oxoethyl)isoquinolin-1-yl]methyl}-2-(trifluoromethyl)benzoate (0.065 g) as a yellow amorphous substance.

$^1$H NMR (600 MHz, CHLOROFORM-d) δ ppm 1.55 (s, 9 H), 3.70 (s, 3 H), 4.02 (s, 2 H), 4.72 (s, 2 H), 7.42 - 8.42 (m, 8 H)

(7) The same procedure as used in Example 1-(6) was carried out using the compound (0.065 g) obtained in Example 7-(6) to give 4-{[4-(2-methoxy-2-oxoethyl)isoquinolin-1-yl]methyl}-2-(trifluoromethyl)benzoic acid (0.057 g) as a colorless solid.

$^1$H NMR (600 MHz, DMSO-$d_6$) δ ppm 3.64 (s, 3 H), 4.30 (br. s., 2 H), 5.02 (br. s., 2 H), 7.67 - 8.65 (m, 8 H)

(8) The same procedure as used in Example 3-(8) was carried out using the compound (0.057 g) obtained in Example 7-(7) to give methyl {1-[4-{[2-(4-chlorophenyl)ethyl]carbamoyl}-3-(trifluoromethyl)benzyl]isoquinolin-4-yl}acetate (0.052 g) as a pale yellow amorphous substance.

$^1$H NMR (600 MHz, CHLOROFORM-d) δ ppm 2.87 (t, J = 6.9 Hz, 2 H), 3.64 - 3.69 (m, 2 H), 3.70 (s, 3 H), 4.02 (s, 2 H), 4.70 (s, 2 H), 5.71 (br. s., 1H), 7.13 - 8.41 (m, 12 H)

(9) The same procedure as used in Example 1-(8) was carried out using the compound (0.052 g) obtained in Example 7-(8) to give the title compound (0.032 g) as a pale yellow solid.

$^1H$ NMR (600 MHz, DMSO-$d_6$) δ ppm 2.76 (t, J = 7.1 Hz, 2 H), 3.36 - 3.42 (m, 2 H), 4.01 (s, 2 H), 4.76 (s, 2 H), 7.22 - 8.49 (m, 13 H)

The following compounds were synthesized as Reference Examples.

[Reference Example 1]

3-[1-(4-{[2-(4-chlorophenyl)ethyl]carbamoyl}benzyl)isoquinolin-4-yl]propanoic acid

[Ka 31]

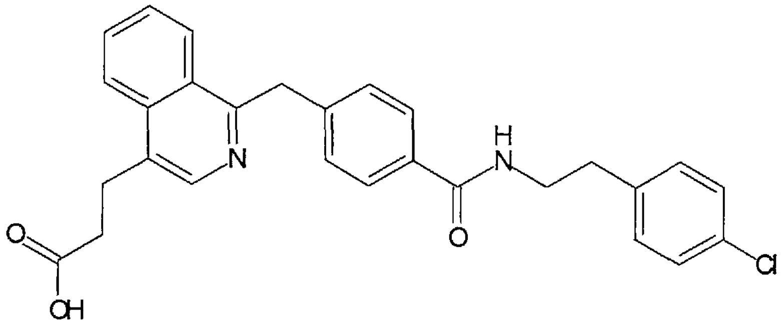

$^1H$ NMR (600 MHz, DMSO-$d_6$) δ ppm 2.65 (t, J = 7.7 Hz, 2 H), 2.80 (t, J = 7.1 Hz, 2 H), 3.24 (t, J = 7.7 Hz, 2 H), 3.40 - 3.46 (m, 2 H), 4.65 (s, 2 H), 7.20 - 8.43 (m, 14 H)

[Reference Example 2]

{[1-(4-{[2-(4-chlorophenyl)ethyl]carbamoyl}benzyl)isoquinolin-4-yl]oxy}acetic acid

[Ka 32]

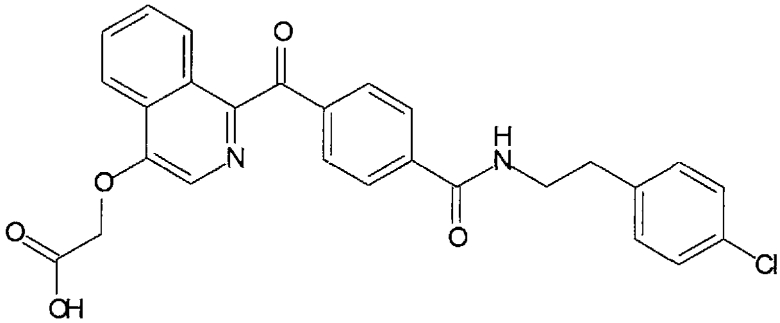

$^1H$ NMR (600 MHz, DMSO-$d_6$) δ ppm 2.86 (t, J = 7.1 Hz, 2 H), 3.48 - 3.53 (m, 2 H), 5.12 (s, 2 H), 7.26 - 8.75 (m, 14 H)

[Reference Example 3]

3-[1-(4-{[2-(4-chlorophenyl)ethyl]carbamoyl}benzyl)isoquinolin-4-yl]-2-methylpropanoic acid

[Ka 33]

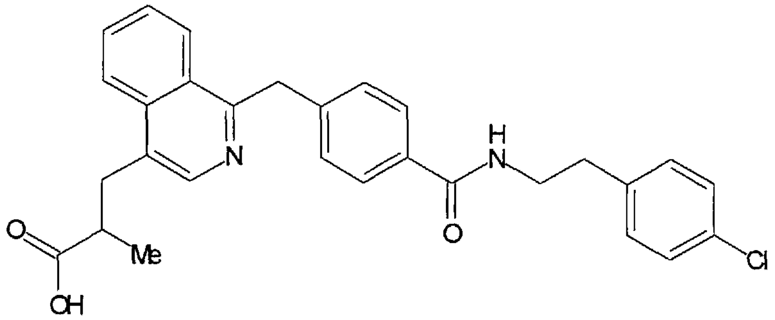

$^1H$ NMR (600 MHz, DMSO-$d_6$) δ ppm 1.12 (d, J = 7.3 Hz, 3 H), 2.71 - 2.77 (m, 1H), 2.80 (t, J = 7.3 Hz, 2 H), 2.99 - 3.04 (m, 1H), 3.28 - 3.35 (m, 1 H), 3.40 - 3.47 (m, 2 H), 4.65 (s, 2 H), 7.21 - 8.44 (m, 14 H)

[Reference Example 4]

3-[1-(4-{[2-(4-chlorophenyl)ethyl]carbamoyl}benzyl)isoquinolin-4-yl]-2,2-dimethylpropanoic acid

[Ka 34]

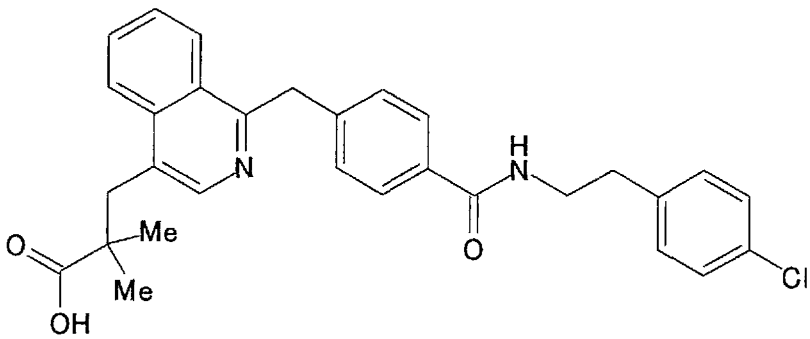

$^1H$ NMR (600 MHz, DMSO-$d_6$) δ ppm 1.12 (s, 6 H), 2.80 (t, J = 7.1 Hz, 2 H), 3.27 (s, 2 H), 3.40 - 3.45 (m, 2 H), 4.65 (s, 2 H), 7.21 - 8.43 (m, 14 H)

[Reference Example 5]

4-[1-(4-{[2-(4-chlorophenyl)ethyl]carbamoyl}benzyl)isoquinolin-4-yl]butanoic acid

[Ka 35]

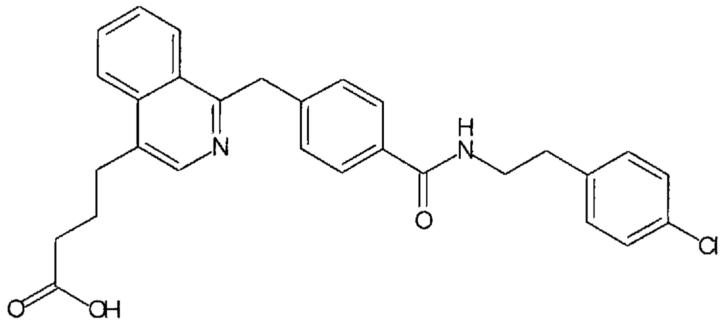

$^1$H NMR (600 MHz, DMSO-d6) δ ppm 1.85 - 1.91 (m, 2 H), 2.34 (t, J = 7.3 Hz, 2 H), 2.80 (t, J = 7.1 Hz, 2 H), 2.98 - 3.02 (m, 2 H), 3.40 - 3.46 (m, 2 H), 4.65 (s, 2 H), 7.20 - 8.43 (m, 14 H)

[Test Example 1] CRTH2 antagonist test

The antagonist activity of the compound of the present invention was considered by using the intracellular calcium ion concentration increase reaction induced when the prostaglandin D2 was added to KB8 cells, which are human cells on which CRTH2 is expressed.

Fluo-4-AM (SIGMA, final concentration: 1 μM) was added to KB8 cells, and the cells were incubated at 37°C for 30 minutes, washed with phosphate buffer (Invitrogen), and then suspended in Hank's balanced salt solution (Invitrogen) containing a reaction buffer solution (10 mM HEPES (Invitrogen), and 1 mM calcium chloride (SIGMA)). The suspension was dispensed in a 96 well plate (Nunc) so that $2\times10^5$ cells/well were placed, and the compound of the present invention and PGD2 (final concentration: 100 nM) were added. The fluorescence intensity thereof was measured over time by using FDSS6000 (Hamamatsu Photonics), and thus the maximum fluorescence intensity value "d" was obtained. The same procedure was carried out in the absence of the compound, and the maximum fluorescence intensity value "e" was obtained; and the same procedure was carried out in the absence of the compound and in the presence of non-labeled PGD2, and the maximum fluorescence intensity value "f' was obtained.
The calcium ion concentration increase inhibition rate of a compound was calculated by the following calculation equation:

$$\text{Inhibitory rate (\%)} = [1- (d-f) / (e-f)] \times 100$$

Furthermore, the CRTH2 antagonist activity of a compound to be tested was calculated as a value ($IC_{50}$ value) exhibiting 50% inhibitory activity with respect to the calcium ion concentration increase in the absence of the compound. That is to say, by using calcium ion concentration increase inhibitory rates of compounds to be tested having various concentrations, the $IC_{50}$ value was calculated according to a dose-dependent inhibition curve analyzed by using XLfit (IDBS) as a data analysis software, and the value was defined as an indicator of the antagonist activity. The test results were as follows: Example 1 ($IC_{50}$ value: 4.4 nM), Example 2 ($IC_{50}$ value: 36 nM), Example 3 ($IC_{50}$ value: 17 nM), Example 4 ($IC_{50}$ value: 15 nM), Example 5 ($IC_{50}$ value: 42 nM), Example 6 ($IC_{50}$ value: 8.7 nM), and Example 7 ($IC_{50}$ value: 14 nM).

Industrial Applicability

The present invention is directed to a compound having a CRTH2 inhibitory activity, which can be used by being incorporated into preventive agents or therapeutic agents for allergic diseases such as asthma, atopic dermatitis, and allergic rhinitis.

**Claims**

1. A compound represented by formula (I):

[Ka. 1]

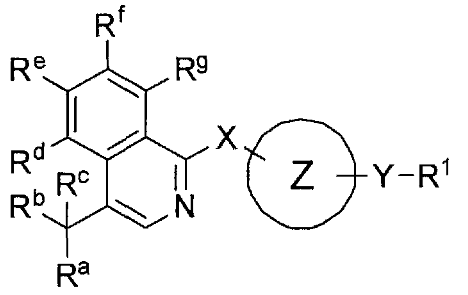

(I)

wherein $R^1$ represents a $C_{1-6}$ alkyl group, a $C_{2-6}$ alkenyl group, a $C_{3-6}$ cycloalkyl group, a $C_{3-6}$ cycloalkenyl group, an adamantyl group, an indanyl group, a tetrahydronaphthyl group, a tetrahydroindolyl group, a tetrahydropyranyl group, a morpholinyl group, a phenyl group, a naphthyl group, or an aromatic heterocyclic group, wherein the phenyl group, the naphthyl group, and the aromatic heterocyclic group may be substituted with 1 to 5 substituent(s) selected from the group consisting of a $C_{1-6}$ alkyl group, a $C_{2-6}$ alkenyl group, a $C_{3-6}$ cycloalkyl group, a halogen atom, a $C_{1-6}$ alkoxy group, a hydroxy group, a $C_{1-6}$ alkylthio group, a $C_{1-6}$ haloalkyl group, a $C_{1-6}$ haloalkoxy group, a $C_{1-6}$ haloalkylthio group, a cyano group, a nitro group, a guanidino group, a $C_{1-6}$ alkylsulfonyl group, a carboxy group, a $C_{2-7}$ alkoxycarbonyl group, a $C_{2-7}$ alkanoyloxy group, a phenyl group, a benzoyl group, a phenoxy group, a pyrrolyl group, a thienyl group, an imidazolyl group, a thiadiazolyl group, a morpholino group, the formula: - $NR^5R^6$, the formula: -$SO_2NR^7R^8$, the formula: -$NR^9SO_2R^{10}$, the formula: -$CONR^{11}R^{12}$, and the formula: -$NR^{13}COR^{14}$, wherein $R^5$, $R^6$, $R^7$, $R^8$, $R^9$, $R^{10}$, $R^{11}$, $R^{12}$, $R^{13}$, and $R^{14}$ each independently represent a hydrogen atom or a $C_{1-6}$ alkyl group;
X represents an oxygen atom, a sulfur atom, the formula: -$CR^hR^i$-, the formula: -CO-, the formula: -$NR^2$-, or formula (II):

[Ka. 2]

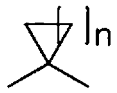

(II)

wherein $R^h$ and $R^i$ each independently represent a hydrogen atom, a $C_{1-6}$ alkyl group, a halogen atom or a $C_{1-6}$ haloalkyl group;
$R^2$ represents a hydrogen atom or a $C_{1-6}$ alkyl group;
n represents an integer of 1 to 4;
Y represents a single bond, the formula: -$NR^3$CO-W-, the formula: -$NR^3$CO-W-O-, the formula: - $NR^3CO_2$-W-, the formula: -$NR^3$-W-, the formula: -$NR^3SO_2$-W-, the formula: -$NR^3CONR^4$-W-, the formula: -$NR^3$CO-W-$NR^4SO_2$-, the formula: -$SO_2NR^3$-W-, the formula: -$CH_2$-W-, the formula: -$CONR^3$-W-, the formula: -$CONR^3$-W-O-, the formula: -$CH_2$-O-W-, the formula: - $CH_2NR^3$-W-, the formula: -$CONR^3$-W-$NR^4$CO-, the formula: -O-W-, or the formula: -O-W-O-,
wherein $R^3$ and $R^4$ each independently represent a hydrogen atom or a $C_{1-6}$ alkyl group, W is a single bond, a $C_{1-6}$ alkylene group, a $C_{2-6}$ alkylene group including a carbon atom that is also a member of a $C_{3-6}$ cycloalkyl ring, a $C_{2-6}$ alkenylene group, or a $C_{3-6}$ cycloalkylene group (provided that, when Y is the formula: -$CONR^3$-W-$NR^4$CO- or the formula: -O-W-O-, W is not a single bond);
Z represents a benzene ring, a pyrimidine ring, or a pyrazine ring;
said benzene ring, pyrimidine ring, and pyrazine ring may be substituted with 1 to 4 substituent(s) selected from the group consisting of a $C_{1-6}$ alkyl group, a $C_{2-6}$ alkenyl group, a $C_{3-6}$ cycloalkyl group, a halogen atom, a $C_{1-6}$ alkoxy group, a hydroxy group, a $C_{1-6}$ alkylthio group, a $C_{1-6}$ haloalkyl group, a $C_{1-6}$ haloalkoxy group, a $C_{1-6}$ haloalkylthio group, a cyano group, a nitro group, a $C_{1-6}$ alkylsulfonyl group, a carboxy group, the formula: -$NR^{22}R^{23}$, the formula: - $SO_2NR^{24}R^{25}$, the formula: -$NR^{26}SO_2R^{27}$, the formula: -$CONR^{28}R^{29}$, and the formula: - $NR^{30}COR^{31}$, wherein $R^{22}$, $R^{23}$, $R^{24}$, $R^{25}$, $R^{26}$, $R^{27}$, $R^{28}$, $R^{29}$, $R^{30}$, and $R^{31}$ each independently represent a hydrogen atom or a $C_{1-6}$ alkyl group (provided that, when Z is an unsubstituted benzene ring, unsubstituted pyrimidine ring, or unsubstituted pyrazine

ring, X is the formula: - $CR^hR^i$-, or formula (II), wherein at least one of $R^h$ and $R^i$ represents a $C_{1-6}$ alkyl group, a halogen atom, or a $C_{1-6}$ haloalkyl group);
$R^a$ represents a carboxy group, a carbamoyl group, a tetrazolyl group, or the formula: - CONHOH;
$R^b$ and $R^c$ each independently represent a hydrogen atom, a halogen atom, or a $C_{1-6}$ alkyl group; and
$R^d$, $R^e$, $R^f$ and $R^g$ each independently represent a hydrogen atom, a halogen atom, a $C_{1-6}$ alkyl group, or a $C_{1-6}$ alkoxy group (provided that the compound is not {1-[2-fluoro-5-(propan-2-yloxy)benzyl] -6,7-dimethoxyisoquinolin-4-yl}acetic acid, 2-[1-(2-fluoro-5-methoxybenzoyl)-6,7-dimethoxyisoquinolin-4-yl)acetamide, {1-[3-fluoro-5-(propan-2-yloxy)benzoyl] -6,7-dimethoxyisoquinolin-4-yl}acetic acid, 2- {1-[3-fluoro-5-(propan-2-yloxy)benzoyl] -6,7-dimethoxyisoquinolin-4-yl}propanoic acid, 2-{1-[2-fluoro-5-(propan-2-yloxy)benzoyl] -6,7-dimethoxyisoquinolin-4-yl}-4-methylpentanoic acid, 2-{1-[2-fluoro-5-(propan-2-yloxy)benzoyl] -6,7-dimethoxyisoquinolin-4-yl}propanoic acid, {1-[2-fluoro-5-(propan-2-yloxy)benzoyl] -6,7-dimethoxyisoquinolin-4-yl} acetic acid, or [6,7-dimethoxy-4-(1H-tetrazol-5-ylmethyl)isoquinolin-1-yl](2-fluoro-5-methoxyphenyl)methanone);
or a pharmaceutically acceptable salt thereof.

**2.** The compound or a pharmaceutically acceptable salt thereof according to claim 1, wherein $R^d$, $R^e$, $R^f$ and $R^g$ each independently represent a hydrogen atom, a halogen atom, a $C_{1-6}$ alkyl group, or a $C_{1-6}$ alkoxy group (except the compound or a pharmaceutically acceptable salt thereof in which both $R^d$ and $R^g$ are hydrogen atoms and both $R^e$ and $R^f$ are $C_{1-6}$ alkoxy groups).

**3.** The compound or a pharmaceutically acceptable salt thereof according to claim 1 or 2, wherein
X is the formula: -$CR^hR^i$-, wherein $R^h$ is a hydrogen atom, a $C_{1-6}$ alkyl group, or a halogen atom;
and $R^1$ is a $C_{1-6}$ alkyl group, or a halogen atom.

**4.** The compound or a pharmaceutically acceptable salt thereof according to any one of claims 1-3, wherein
Z is a benzene ring substituted with a $C_{1-6}$ alkyl group, a halogen atom, a $C_{1-6}$ alkoxy group, or a $C_{1-6}$ haloalkyl group.

**5.** The compound or a pharmaceutically acceptable salt thereof according to any one of claims 1-4, wherein
$R^1$ is a phenyl group, which may be substituted with a halogen atom;
Y is the formula: -$CONR^3$-W-;
W is a $C_{1-6}$ alkylene group;
$R^a$ is a carboxy group;
$R^b$ and $R^c$ are each a hydrogen atom, and
$R^d$, $R^e$, $R^f$ and $R^g$ are each a hydrogen atom.

**6.** A preventive or a remedy for asthma, atopic dermatitis and allergic rhinitis, comprising the compound or a pharmaceutically acceptable salt thereof according to any one of claims 1-5 as an active ingredient.

## INTERNATIONAL SEARCH REPORT

International application No.
PCT/JP2011/064208

A. CLASSIFICATION OF SUBJECT MATTER
*C07D217/18*(2006.01)i, *A61K31/472*(2006.01)i, *A61P11/02*(2006.01)i, *A61P11/06*(2006.01)i, *A61P17/04*(2006.01)i, *A61P37/08*(2006.01)i, *A61P43/00* (2006.01)i, *C07D217/20*(2006.01)i
According to International Patent Classification (IPC) or to both national classification and IPC

B. FIELDS SEARCHED
Minimum documentation searched (classification system followed by classification symbols)
C07D217/18, A61K31/472, A61P11/02, A61P11/06, A61P17/04, A61P37/08, A61P43/00, C07D217/20

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CAplus(STN), REGISTRY(STN), WPI

C. DOCUMENTS CONSIDERED TO BE RELEVANT

| Category* | Citation of document, with indication, where appropriate, of the relevant passages | Relevant to claim No. |
|---|---|---|
| X<br>A | WO 2004/101528 A2 (HOFFMANN LA ROCHE & CO. AG. F),<br>25 November 2004 (25.11.2004),<br>examples; claims<br>& US 2004/259910 A1 & EP 1631551 A2<br>& US 7067529 B2 & JP 2006-528218 A | 1,3-4<br>2,5-6 |
| A | WO 2009/060209 A1 (ARGENTA DISCOVERY LTD., GB),<br>14 May 2009 (14.05.2009),<br>entire text<br>(Family: none) | 1-6 |
| A | EP 30861 A2 (PFIZER INC.),<br>24 June 1981 (24.06.1981),<br>compound III<br>& JP 56-92871 A & US 4283539 A | 1-6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 July, 2011 (08.07.11) | 19 July, 2011 (19.07.11) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2011/064208

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 2010/074244 A1 (TAISHO PHARMACEUTICAL CO., LTD.), 01 July 2010 (01.07.2010), entire text (Family: none) | 1-6 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005019171 A
- WO 2005115382 A
- WO 2004096777 A
- WO 2004101528 A


### Non-patent literature cited in the description


- **NAGATA et al.** *J. Immunol.,* 1999, vol. 162, 1278
- **DEL PRETE et al.** *Allergy,* 1992, vol. 47, 450
- **POPE et al.** *J. Allergy. Clin. Immunol.,* 2001, vol. 108, 594
- **MIN et al.** *Curr. Opin. Hematol.,* 2008, vol. 15, 59
- **BROIDE et al.** *J. Allergy. Clin. Immunol.,* 2001, vol. 108 (2), 65
- **ABE et al.** *Gene,* 1999, vol. 227, 71
- **HIRAI et al.** *J. Exp. Med.,* 2001, vol. 193, 225
- **SHIRAISHI et al.** *J. Pharmacol. Exp. Ther.,* 2005, vol. 312, 954
- **SATOH et al.** *J. Immunol.,* 2006, vol. 177, 2621
- **KANO et al.** *Am. J. Rhinol.,* 2006, vol. 20, 342
- **P. G M. WUTS ; T. GREEN.** Protective Groups in Organic Synthesis. 1999
- **P. G. M. WUTS ; T. GREEN.** Protective Groups in Organic Synthesis. 1999